# EUROPEAN PATENT APPLICATION

(11) **EP 3 461 396 A2**
(43) Date of publication of application: **03.04.2019**
(21) Application number: 18196818.1
(22) Date of filing: 26.09.2018
(51) Int. Cl.: A61B 3/08

(54) **OPHTHALMIC DEVICE**

(30) Priority: 29.09.2017 JP 2017191478; 10.10.2017 JP 2017197205
(71) Applicant: Nidek Co., Ltd., Gamagori-shi, Aichi, 443-0038 (JP)
(72) Inventor: TAKII, Michihiro, Gamagori-shi, Aichi 443-0038 (JP); TACHIBANA, Sasagu, Gamagori-shi, Aichi 443-0038 (JP); TANAKA, Masaki, Gamagori-shi, Aichi 443-0038 (JP); TOCHIKUBO, Yujiro, Gamagori-shi, Aichi 443-0038 (JP)
(74) Representative: Viering, Jentschura & Partner mbB Patent- und Rechtsanwälte

(57) **Abstract**

An ophthalmic device includes an anterior ocular segment photographing unit (50), a fixation target display unit, and a control unit (71). The anterior ocular segment photographing unit captures an anterior ocular segment image of a left eye and a right eye of the subject. The fixation target display unit displays a fixation target with respect to the at least one of the left eye and the right eye of the subject. The control unit measures an eye position of the at least one of the left eye and the right eye of the subject by processing the anterior ocular segment image captured by the anterior ocular segment photographing unit. The control unit generates eye position state information indicating a state of the eye position of the subject, based on measurement results of the eye position at at least two timings including a post-switch timing and a pre-switch timing.

## Description

### TECHNICAL FIELD

The present disclosure relates to an ophthalmic device used to perform an examination of a visual function of a subject.

### BACKGROUND

Various ophthalmic devices are known that are used to perform an examination of a visual function of a subject. For example, in a subjective optometry device disclosed in Japanese Laid-Open Patent Publication No. H5-176893, a corrective optical system that can correct refractivity is placed separately in front of an eye of a subject, and an examination target is projected onto the ocular fundus of the eye of the subject via the corrective optical system. A tester receives a response of the subject and adjusts the corrective optical system until the subject can see the target properly, and thus measures the refractive power of the subject's eye. In a subjective optometry device disclosed in U.S. Patent No. 3874774, by forming, in front of the eye of the subject, an examination target image that has passed through a corrective optical system, the refractive power is measured without placing the corrective optical system. In addition to the refractive power, the visual function of the subject includes a function relating to an eye position, which is a direction in which the eye is facing.

### SUMMARY

In known technology, it is difficult to objectively examine a state relating to an eye position. For example, one state of the eye position is heterophoria. As a method for examining the presence or absence of heterophoria, a cover-uncover test is known, for example. In the cover-uncover test, a cover covering the visual field of one eye is removed, and the presence or absence of heterophoria is examined by the tester visually verifying movement of the eye at that time. In this method, as well as requiring skill by the tester, it is difficult to quantitatively show an examination result. Although a method is known in which a prism is added to perform a heterophoria examination using an examination device, this is a subjective examination. Thus, with these methods, depending on the response of the subject, there may potentially be cases in which the examination result is not stable or the examination requires time.

A typical object of the present disclosure is to provide an ophthalmic device capable of appropriately performing an objective examination of a state relating to an eye position.

An ophthalmic device provided by typical embodiments of the present disclosure is an ophthalmic device that performs an examination of a visual function of a subject, the ophthalmic device including an anterior ocular segment photographing unit that captures an anterior ocular segment image of at least one of a left eye and a right eye of the subject, a fixation target display unit that displays a fixation target with respect to the at least one of the left eye and the right eye of the subject, and a processor that controls the ophthalmic device, wherein the processor measures an eye position of the at least one of the left eye and the right eye of the subject by processing the anterior ocular segment image captured by the anterior ocular segment photographing unit, and generates eye position state information based on measurement results of the eye position of an examination target eye at at least two timings including a post-switch timing and a pre-switch timing, the eye position state information indicating a state of the eye position of the subject, the post-switch timing being a timing after a display switch timing at which display and non-display of the fixation target with respect to the at least one of the left eye and the right eye of the subject is switched, and the pre-switch timing being a timing before the display switch timing.

According to the ophthalmic device according to the present disclosure, an objective examination of a state relating to an eye position can be appropriately performed.

An ophthalmic device exemplified in the present disclosure includes an anterior ocular segment photographing unit, a fixation target display unit, and a processor. The anterior ocular segment photographing unit captures an anterior ocular segment image of at least one of a left eye and a right eye of the subject. The fixation target display unit displays a fixation target with respect to the at least one of the left eye and the right eye of the subject, thus causing the at least one eye to pay attention to the fixation target. The processor measures an eye position of the at least one of the left eye and the right eye of the subject by processing the anterior ocular segment image captured by the anterior ocular segment photographing unit. The eye position is a direction in which the eye is facing. The processor generates eye position state information based on measurement results of the eye position of an examination target eye at at least two timings including a post-switch timing and a pre-switch timing, the eye position state information indicating a state of the eye position of the subject, the post-switch timing being a timing after a display switch timing at which display and non-display of the fixation target with respect to the at least one of the left eye and the right eye is switched, and the pre-switch timing being a timing before the display switch timing.

According to the ophthalmic device exemplified in the present disclosure, the eye position state information showing the state of the eye position before and after the display switch timing of the fixation target is obtained on the basis of the anterior ocular segment image. Thus, the objective examination of the state relating to the eye position can be appropriately performed.

A specific method to measure the eye position from the anterior ocular segment image may be selected as appropriate. For example, the processor may detect a pupil position of the eye by processing the anterior ocular segment image, and may measure the eye position on the basis of the detected pupil position. In this case, the pupil position may be any portion of the pupil. For example, at least one of a center position of the pupil, a position of an outer peripheral edge of the pupil, and the like may be detected as the pupil position. The eye position may be measured on the basis of a distance between the pupils of the left eye and the right eye.

The processor may detect a corneal apex position of the eye by processing the anterior ocular segment image, and may measure the eye position on the basis of the detected corneal apex position. The corneal apex position may be detected, for example, by detecting a visual target image projected onto the eye, and detecting the corneal apex position on the basis of the position of the detected visual target image. The eye position may be measured on the basis of a distance between the corneal apexes of the left eye and the right eye.

The processor may detect both the pupil position and the corneal apex position of the eye by processing the anterior ocular segment image, and may measure the eye position on the basis of the detected pupil position and corneal apex position. In this case, for example, the eye position may be measured on the basis of a displacement between a pupil center position and the corneal apex position. An absolute value of the eye position need not necessarily be measured, and it is sufficient that a determination can be made at least in relation to changes in the eye position on the basis of measurement results.

The processor may generate, as the eye position state information, at least data of an eye position change graph showing a relationship between time and the eye position of the examination target eye after the display switch timing. In this case, changes over time in the eye position of the examination target eye resulting from the switching between the display and the non-display of the fixation target can be appropriately ascertained using the eye position change graph. By viewing the eye position change graph, the tester also can perform a diagnosis regarding the presence/absence of heterophoria and the like. However, the ophthalmic device may generate only a displacement amount of the eye position (to be described below), without generating the eye position change graph.

The processor may display the eye position change graph on a display unit. In this case, the processor may continuously measure the eye position during the examination of the eye position of the examination target eye, and may display the eye position change graph on the display unit in real time during the examination. The processor may generate data of the eye position change graph after the examination, based on the anterior ocular segment images captured continuously during the examination.

The eye position change graph may be a graph in which a plurality of eye position measurement results are plotted for each of measurement timings, or may be a graph of an approximation curve generated on the basis of the plurality of eye position measurement results.

The processor may generate, as the eye position state information, a displacement amount between a measurement result of the eye position at at least one pre-switch timing before the display switch timing of the fixation target and a measurement result of the eye position at at least one post-switch timing after the display switch timing.

In this case, the displacement amount of the eye position resulting from the switching of the display and the non-display of the fixation target is quantitatively ascertained. Thus, a diagnosis relating to the eye position for the presence/absence of heterophoria, for example, can be more appropriately performed. However, the ophthalmic device may generate only the eye position change graph, without generating the displacement amount of the eye position.

The processor may generate the displacement amount based on a measurement result of the eye position at at least one post-switch timing after a reference timing, which is a timing at which a stand-by time period elapses from the display switch timing of the fixation target. When there is heterophoria, there is a tendency for the eye position to change gradually after the switching between the display and the non-display of the fixation target, and to become stable as time elapses. Thus, if the measurement results of the eye position immediately after the switching between the display and the non-display of the fixation target are used, the generated displacement amount of the eye position is likely to be an inappropriate value. In contrast to this, by using the measurement results of the eye position after the elapse of a stand-by time period, the accuracy of the generated displacement amount of the eye position can be improved. A length of the stand-by time period may be set to a length that is equal to or more than a time period required for the changes in the eye position of the examination target eye to become stable, after the switching between the display and the non-display of the fixation target. In this case, the displacement amount of the eye position can be calculated on the basis of the eye position in a stable state. In the ophthalmic device, one or a plurality of stand-by time periods may be set in advance.

The processor may receive input of a command specifying the reference timing, and may set the reference timing in accordance with the input command. In this case, a tester can set a desired timing as the reference timing, in accordance with various conditions (one's own experience, a state of the subject's eye, and so on, for example).

A specific method to receive the command specifying the reference timing may be selected as appropriate. For example, the processor may receive the command specifying the reference timing by causing a user (a tester, for example) to specify the length of the stand-by time period from the display switch timing of the fixation target to the reference timing. When the command specifying the reference timing is received in real time during the examination, the processor may set a timing input by the specifying command as the reference timing.

The processor may receive input of the command specifying the reference timing in a state in which the eye position change graph is displayed on the display unit. In this case, the tester can specify an appropriate timing as the reference timing while understanding the state of changes in the eye position using the eye position change graph. Note that the display of the eye position change graph and the setting of the reference timing may be performed in real time during the examination, or may be performed after the examination. When the display of the eye position change graph and the setting of the reference timing are performed after the examination, the processor may receive the input of the command specifying the reference timing by causing the user to specify a desired timing on the eye position change graph.

When the specified timing is set as the reference timing, a time period may be set in which the setting of the reference timing is prohibited. For example, a period from the switching between the display and the non-display of the fixation target to the elapse of a minimum stand-by time period required for the changes in the eye position of the examination target eye to become stable (one second or more, for example) may be designated as the period in which the setting of the reference timing is prohibited. In this case, the tester can specify the reference timing only after the elapse of the minimum stand-by time period that is required for the changes in the eye position to become stable. Thus, the displacement amount of the eye position can be more likely to be calculated on the basis of the eye position in the stable state.

The processor may detect a timing at which the eye position of the examination target eye becomes stable after the display switch timing, based on measurement results of the eye position at a plurality of the post-switch timings, and may set the detected timing as the reference timing. In this case, the appropriate reference timing can be automatically set by the ophthalmic device.

A specific method to detect the timing at which the eye position becomes stable may be selected as appropriate.

For example, the processor may detect fluctuations in the measurement results, from the measurement results of the eye position at a plurality of the post-switch timings. The processor may detect, as the timing at which the eye position becomes stable, a timing at which the detected fluctuation becomes equal to or less than a threshold value. For example, at least one of a standard deviation of the plurality of measurement results within a unit period of time, a difference between a maximum value and a minimum value of the plurality of measurement results within the unit period of time, and a frequency or the like of the varying measurement results may be used as the fluctuation in the measurement results. In this case, the timing at which the eye position becomes stable can be appropriately detected on the basis of the fluctuations in the measurement results. The processor may output, as the eye position state information, information about the fluctuation in the detected measurement results. In this case, the tester can more appropriately determine the state of the eye position of the examination target eye.

The processor may generate data of an approximation curve showing a relationship between the eye position and time, from the measurement results of the eye position at the plurality of post-switch timings. The processor may detect, as the timing at which the eye position becomes stable, a timing at which an inclination of the approximation curve becomes equal to or less than a threshold value. In this case, the timing at which the eye position becomes stable can be appropriately detected on the basis of the approximation curve showing the fluctuations in the eye position. As described above, the processor may output data of the generated approximation curve as the eye position state information.

The ophthalmic device may include an objective measurement unit. The objective measurement unit may objectively measure an optical characteristic of the examination target eye by emitting measurement light onto the examination target eye and receiving the reflected light. The processor may detect a timing at which the eye position of the examination target eye becomes stable after the display switch timing, based on measurement results of the optical characteristic of the examination target eye measured at a plurality of the post-switch timings, and may set the detected timing as the reference timing. When the eye position becomes stable, the optical characteristic of the examination target eye is also likely to become stable. Thus, by setting the timing at which the optical characteristic of the examination target eye becomes stable as the reference timing, the appropriate reference timing can be automatically set.

A specific method to detect the timing at which the eye position becomes stable on the basis of the measurement results of optical characteristic may also be selected as appropriate.

For example, the processor may detect, from the measurement results of the optical characteristic at the plurality of post-switch timings, fluctuations in the measurement results (at least one of a standard deviation of the plurality of measurement results within a unit period of time, a difference between a maximum value and a minimum value of the plurality of measurement results within the unit period of time, and a frequency or the like of the varying measurement results). The processor may detect, as the timing at which the eye position become stable, a timing at which the detected fluctuation becomes equal to or less than a threshold value. The processor may output information about the fluctuations in the measurement results of the optical characteristic.

The processor may generate data of an approximation curve showing the relationship between the optical characteristic and time, from the measurement results of the optical characteristic at the plurality of post-switch timings. The processor may detect, as the timing at which the eye position becomes stable, a timing at which an inclination of the approximation curve becomes equal to or less than a threshold value. The processor may output the data of the approximation curve showing the fluctuations in the measurement results of the optical characteristic.

For example, the refractivity or the like of the examination target eye may be used as the optical characteristic used to detect the stability of the eye position. The stability of the eye position may be detected using objective measurement results other than the refractivity (the size of the pupil of the examination target eye, for example). Further, the stability of the eye position may be detected on the basis of both the measurement results of the eye position and the measurement results of the optical characteristic.

When the timing at which the eye position becomes stable (hereinafter referred to as an "eye stability timing") is detected on the basis of the measurement results of at least one of the eye position and the optical characteristic, the processor may output information about the time period up to the eye stability timing from the display switch timing of the fixation target. In this case, the tester can more appropriately ascertain the state of the eye position of the examination target eye.

The processor may identify, from the plurality of measurement results of the eye position measured at the plurality of post-switch timings after the reference timing, an average value, a median value between a maximum value and a minimum value, or a most frequent value that is a most frequently measured value. The processor may calculate, as the eye position state information, a displacement amount between the identified value and the measurement results of the eye position at the pre-switch timing. In this case, the displacement amount of the eye position is calculated while an influence of the fluctuations in the eye position is further suppressed.

An average value, a median value, or a most frequent value of the plurality of measurement results of the eye position of the pre-switch timing before the display switch timing of the fixation target may be used. The displacement amount of the eye position may be calculated on the basis of at least one of an eye position measurement result at a single post-switch timing and the eye position measurement result at a single pre-switch timing.

From the plurality of eye position measurement results at the plurality of pre-switch timings, and the plurality of eye position measurement results at the plurality of post-switch timings, the processor may identify a first most frequent value that is the value most frequently measured before the display switch timing and a second most frequent value that is the value most frequently measured after the display switch timing. The processor may calculate a displacement amount between the first most frequent value and the second most frequent value as the eye position state information. In this case, the displacement amount between the eye position that is in the stable state before the display switch timing of the fixation target and the eye position that is in the stable state after the display switch timing of the fixation target can be appropriately calculated. Thus, the accuracy of the generated (calculated) displacement amount of the eye position can be improved.

The processor may acquire the display switch timing at which the display and the non-display of the fixation target is switched, and may generate the eye position state information on the basis of the acquired display switch timing. In this case, the eye position state information with a high degree of accuracy can be generated on the basis of the display switch timing.

A specific method to acquire the display switch timing may be selected as appropriate. For example, the processor may acquire the display switch timing by receiving an input of a command from the user specifying the display switch timing. Further, when the display and the non-display of the fixation target is switched by the insertion and removal of a visible light shielding member, the processor may acquire the display switch timing by detecting, using image processing or the like, a point in time at which a strength of non-visible light received by the anterior ocular segment photographing unit changes due to the insertion or removal of the visible light shielding member. The ophthalmic device may be provided with a sensor that detects that the visible light shielding member has been inserted or removed. Further, when switching means (a display capable of displaying the fixation target, or an actuator that drives a visual target plate including the fixation target, for example) for switching the display and non-display of the fixation target is provided, the processor may acquire a point in time at which the display and the non-display of the fixation target is switched by the switching means, as the display switch timing.

The anterior ocular segment photographing unit may capture the anterior ocular segment image of the left eye and the right eye of the subject. The processor may measure the eye position of each of the left eye and the right eye of the subject by processing the anterior ocular segment image captured by the anterior ocular segment photographing unit. The processor may generate the eye position state information indicating the state of the eye position of the subject, based on measurement results of the eye position of each of the left eye and the right eye at the at least two timings including the pre-switch timing and the post-switch timing.

In this case, the eye position state information indicating the state of the eye position of the subject is generated on the basis of the anterior ocular segment image of the left eye and the right eye before the display switch timing of the fixation target, and the anterior ocular segment image of the left eye and the right eye after the display switch timing. Thus, in comparison to a case in which the eye position state information is generated on the basis of the anterior ocular segment image of one of the left eye and the right eye, the objective examination of the state relating to the eye position can be more appropriately performed. For example, there is a case in which the processor can generate the eye position state information that cannot be obtained with the anterior ocular segment image of one of the left eye and the right eye only. There is a case in which the processor can generate the eye position state information with a high degree of accuracy in comparison to using the anterior ocular segment image of one of the left eye and the right eye only.

When the display of the fixation target with respect to one of the eyes is switched to the non-display, from a state in which the fixation target is being displayed with respect to both the left eye and the right eye, the processor may generate (calculate) a displacement amount between measurement results of the eye position before and after the display switch timing, for each of an eye for which the display of the fixation target is switched to the non-display (a switched eye) and an eye on the opposite side to the switched eye (a non-switched eye). In this case, various determinations relating to the eye position of the subject can be appropriately performed.

For example, before and after the display switch timing, when the displacement amount of the eye position of the non-switched eye is equal to or greater than a threshold value, and the displacement amount of the eye position of the switched eye is equal to or greater than a threshold value, it may be determined that the subject has strabismus, and that the strabismus is concomitant strabismus. Before and after the display switch timing, when the displacement amount of the eye position of the non-switched eye is equal to or greater than the threshold value, and the displacement amount of the eye position of the switched eye is less than the threshold value, it may be determined that the subject has strabismus and that the strabismus is non-comitant strabismus.

When the subject has strabismus, the processor may detect a movement direction of the eye position, from the measurement results of the eye position before and after the display switch timing, and may generate the eye position state information showing a deviation direction (divergent strabismus, convergent strabismus, hypertropia, hypotropia or the like, for example) of the deviated eye on the basis of the detected movement direction. In this case, the deviation direction of the deviated eye can be easily ascertained. The processor may detect the deviation direction of the eye position on the basis of the movement direction of the eye position of the non-switched eye in the examination when the strabismus is detected. When the strabismus is concomitant strabismus, the processor may detect the deviation direction of the eye position on the basis of the movement direction of the eye position of the switched eye in the examination when the strabismus is detected.

Before and after the display switch timing, when the displacement amount of the eye position of the non-switched eye is less than the threshold value, and the displacement amount of the eye position of the switched eye is equal to or greater than the threshold value, it may be determined that heterophoria is present in the switched eye.

The processor may generate the eye position state information indicating at least one of presence/absence of strabismus of the subject and whether the strabismus is concomitant strabismus, based on a displacement amount of the eye position of the switched eye and a displacement amount of the eye position of the non-switched eye. In this case, a determination relating to the strabismus of the subject can be easily performed, using the eye position state information automatically generated by the ophthalmic device.

As described above, when the displacement amount of the eye position of the non-switched eye is equal to or greater than the threshold value, and the displacement amount of the eye position of the switched eye is equal to or greater than the threshold value, the processor may determine that the subject has strabismus and that the strabismus is concomitant strabismus. When the displacement amount of the eye position of the non-switched eye is equal to or greater than the threshold value, and the displacement amount of the eye position of the switched eye is less than the threshold value, the processor may determine that the subject has strabismus and that the strabismus is non-comitant strabismus. The processor may determine only the presence/absence of strabismus, or may determine whether the strabismus is concomitant strabismus.

The processor may generate the eye position state information indicating presence/absence of heterophoria of the switched eye, based on a displacement amount of the eye position of the switched eye and a displacement amount of the eye position of the non-switched eye. In this case, the presence/absence of the heterophoria of the switched eye can be easily ascertained using the eye position state information automatically generated by the ophthalmic device. As described above, when the displacement amount of the eye position of the non-switched eye is less than the threshold value and the displacement amount of the eye position of the switched eye is equal to or greater than the threshold value, the processor may determine that the switched eye has heterophoria.

The processor need not necessarily generate at least one of information indicating the presence/absence of strabismus of the subject and information indicating the presence/absence of heterophoria of the switched eye. For example, the processor may display the displacement amount of the eye position of the switched eye and the displacement amount of the eye position of the non-switched eye before and after the display switch timing on the display unit. In this case, the user (a doctor, for example) can appropriately determine the presence/absence of strabismus, whether the strabismus is concomitant strabismus, and the presence/absence of heterophoria of the switched eye, by viewing the two displayed displacement amounts of the eye positions. The displacement amount of the eye position of the switched eye and the displacement amount of the eye position of the non-switched eye may be notified to the user using voice or the like.

When, from a state in which the fixation target is being displayed with respect to both the left eye and the right eye, the display of the fixation target with respect to one of the eyes is switched to the non-display, the processor may generate (calculate) a displacement amount between the measurement results of the eye position of the non-switched eye before and after the display switch timing. Specifically, the processor may generate only the displacement amount of the eye position of the non-switched eye, without generating the displacement amount of the eye position of the switched eye. In this case also, the presence/absence of the strabismus of the subject can be appropriately determined on the basis of the generated displacement amount of the eye position. The processor may display the displacement amount of the eye position of the non-switched eye on the display unit, or may notify the displacement amount to the user using voice or the like. The processor may generate the eye position state information indicating that the subject has strabismus, based on the displacement amount of the eye position of the non-switched eye.

When, from the state in which the fixation target is being displayed with respect to both the left eye and the right eye, the display of the fixation target with respect to one of the eyes is switched to the non-display, the processor may generate (calculate) the displacement amount between the measurement results of the eye position of the switched eye before and after the display switch timing. Specifically, the processor may generate only the displacement amount of the eye position of the switched eye, without generating the displacement amount of the eye position of the non-switched eye. For example, when it is known in advance that the subject does not have constant strabismus, it can be determined whether the switched eye has heterophoria by referring only to the displacement amount of the eye position the switched eye. When it is known in advance that the subject has strabismus, it can be determined whether the strabismus is concomitant strabismus or non-comitant strabismus by referring only to the displacement amount of the eye position of the switched eye. The processor may display the displacement amount of the eye position of the switched eye on the display unit, or may notify the displacement amount to the user using voice or the like. On the basis of the displacement amount of the eye position of the switched eye, the processor may generate the eye position state information indicating whether the strabismus is concomitant strabismus or non-comitant strabismus or indicating whether the switched eye has heterophoria. In this case, the processor may generate the eye position state information while referring to the information indicating the presence/absence of strabismus of the subject.

When, from a state in which the fixation target is being displayed with respect to one of the left eye and the right eye, the non-display of the fixation target is switched to the display with respect to the other eye, the processor may generate a displacement amount of measurement results of the eye position before and after the display switch timing, for each of an eye for which the non-display of the fixation target is switched to the display (a switched eye) and an eye on the opposite side to the switched eye (a non-switched eye). In this case, various determinations relating to the eye position of the subject can be appropriately performed.

For example, when the subject has strabismus, when at least the displacement amount of the eye position of the non-switched eye is less than the threshold value, it may be determined that the strabismus is alternating strabismus. When the strabismus is concomitant strabismus, when the displacement amount of the eye position of the switched eye before and after the display switch timing and the displacement amount of the eye position of the non-switched eye are both less than the threshold value, it may be determined that the strabismus is alternating strabismus. In contrast, when the subject has strabismus, when at least the displacement amount of the eye position of the non-switched eye is equal to or greater than the threshold value, it may be determined that the strabismus is uniocular strabismus. When the strabismus is concomitant strabismus, when the displacement amount of the eye position of the switched eye and the displacement amount of the eye position of the non-switched eye before and after the display switch timing are both equal to or greater than the threshold value, it may be determined that the strabismus is uniocular strabismus. The control unit may generate the eye position state information indicating whether the strabismus is alternating strabismus or uniocular strabismus on the basis of the displacement amount of the eye position of the switched eye and the displacement amount of the eye position of the non-switched eye before and after the display switch timing.

The determination of the alternating strabismus and the uniocular strabismus can be more appropriately performed by using both the displacement amount of the eye position of the switched eye and the displacement amount of the eye position of the non-switched eye. However, the determination of the alternating strabismus and the uniocular strabismus may be performed on the basis of the displacement amount of the eye position of one of the switched eye and the non-switched eye.

The processor may generate, as the eye position state information, relative information in which the measurement results of the eye position of the left eye is compared with the measurement results of the eye position of the right eye. In this case, the user can appropriately ascertain a relative relationship between the left and right eye positions. For example, the processor may generate, as the eye position state information, a difference between a displacement amount in the measurement results of the eye position of the left eye and a displacement amount in the measurement results of the eye position of the right eye before the display switch timing and after the display switch timing. In this case, the user can appropriately ascertain the state of the left and right eye positions on the basis of the difference between the left and right displacement amounts of the eye positions. The processor may compare other measurement results using the left eye and the right eye. For example, the processor may calculate the displacement amount of the eye position by comparing the left and right eye position measurement results at a given timing (a timing equal to or longer than the elapse of the stand-by time period from the display switch timing, for example).

The processor may generate, as the eye position state information, at least data of an eye position change graph showing a relationship between time and the eye position of each of the left eye and the right eye after the display switch timing. In this case, changes over time in the eye position of each of the left eye and the right eye resulting from the switching between the display and the non-display of the fixation target can be appropriately ascertained using the eye position change graph. The user can also ascertain various states relating to the eye position (the presence/absence of heterophoria, the presence/absence of strabismus, the type of strabismus, for example) by viewing the eye position change graph.

The processor may display the eye position change graph on the display unit. In this case, the processor may continuously measure the eye position during the examination of the eye position of the examination target eye, and display the eye position change graph in real time on the display unit during the examination. The processor may generate the data of the eye position change graph after the examination, based on the anterior ocular segment images captured continuously during the examination.

The eye position change graph may be the graph in which the plurality of eye position measurement results are plotted for each of the measurement timings, or may be the graph of the approximation curve generated on the basis of the plurality of eye position measurement results. When the eye position change graph is displayed on the display unit, the processor may display the eye position change graphs for each of the left eye and the right eye side by side, or may display the approximation curve or the like showing the changes over time of the eye position of each of the left eye and the right eye in a single graph.

The fixation target display unit may be configured to change a position of the displayed fixation target. When the subject has strabismus, the processor may generate, as the eye position state information, information indicating a change amount in measurement results of the eye position of an eye that is deviated with respect to the fixation target when the position of the displayed fixation target is changed. In this case, it can be appropriately determined whether the strabismus is concomitant strabismus or non-comitant strabismus, on the basis of the generated change amount.

The examination as to whether there is strabismus may be performed a plurality of times while changing the position of the fixation target. In this case, the user can determine whether the strabismus is intermittent strabismus or constant strabismus, for example. When the strabismus is alternating strabismus, the user can ascertain the eye position when the deviated eye alternates.

The anterior ocular segment photographing unit may capture the anterior ocular segment image by receiving non-visible light. The display and the non-display of the fixation target may be switched by inserting and removing the visible light shielding member from in front of the eye. The visible light shielding member allows at least part of the non-visible light received by the anterior ocular segment photographing unit to pass through, and blocks the visible light that displays the fixation target.

In this case, by using the non-visible light that passes through the visible light shielding member, the anterior ocular segment photographing unit can appropriately photograph the anterior ocular segment throughout the period from before to after the display switch timing of the fixation target, even when measuring the eye position of the eye for which the display and the non-display of the fixation target is switched. When the display of the fixation target is stopped, the visible light shielding member is inserted in front of the eye. Thus, in contrast to a case in which the display of the fixation target is blocked inside the ophthalmic device, the possibility is low that the subject's eye pays attention to a part of the device after the display is stopped. As a result, after the display of the fixation target is stopped, the vision fixation can be appropriately released.

The method to switch between the display and the non-display of the fixation target may be changed. For example, the ophthalmic device may control a display that is capable of displaying the fixation target, and may switch between the display and the non-display of the fixation target by switching between display and erasure of the fixation target on the display. Further, the ophthalmic device may switch between the display and the non-display of the fixation target by driving a visual target plate on which the fixation target is provided, using an actuator (a motor, for example). The ophthalmic device may switch between the display and the non-display of the fixation target using a polarization element that is provided on a display optical path of the fixation target. In place of inserting and removing the visible light shielding member in front of the eye, the display and the non-display of the fixation target may be switched by inserting and removing a semi-transparent member, which allows only a part of the visible light to pass through, in front of the eye. Even when the semi-transparent member is inserted in front of the eye, the fixation target seen by the subject's eye becomes unfocused, and thus, the display and the non-display of the fixation target can be appropriately switched.

The processor may cause data of the anterior ocular segment image used in the measurement of the eye position to be saved in a storage device. As well as receiving input of a command specifying a photography timing of the anterior ocular segment image photographed in the past, the processor may display, on the display unit, the anterior ocular segment image photographed at the specified photography timing. In this case, the tester can easily compare the eye position measurement result of a desired timing with the anterior ocular segment image used in that measurement of the eye position. Thus, for example, when there is a question about the measurement results of the eye position, the tester can perform an appropriate determination by verifying the anterior ocular segment image used in the measurement of the eye position.

The ophthalmic device may adopt only some of the plurality of technologies illustrated in the present disclosure. For example, the ophthalmic device may generate the data of the eye position change graph showing the relationship between the eye position and time irrespective of the display and the non-display of the fixation target. More specifically, the ophthalmic device may generate the eye position change graph during the display of the fixation target, and may generate the eye position change graph after a predetermined period of time has elapsed after the display of the fixation target is stopped. In this case also, the tester can appropriately ascertain the changes over time in the eye position, using the eye position change graph.

The ophthalmic device may be realized in the following manner. An ophthalmic device used to perform an examination of a visual function of a subject, the ophthalmic device comprising an anterior ocular segment photographing unit that captures an anterior ocular segment image of at least one of a left eye and a right eye of the subject, a fixation target display unit that displays a fixation target with respect to the at least one of the left eye and the right eye of the subject, and a processor that controls the ophthalmic device, wherein the processor measures an eye position of the at least one of the left eye and the right eye of the subject at a plurality of timings, by processing the anterior ocular segment image captured by the anterior ocular segment photographing unit, and generates data of an eye position change graph showing a relationship between the measured eye positions and time.

### BRIEF DESCRIPTION OF THE DRAWINGS

Fig. 1 is an external perspective view of an ophthalmic device 1.
Fig. 2 is a diagram showing a configuration of a left eye measurement unit 7L.
Fig. 3 is an overall configuration diagram of an interior of the ophthalmic device 1 of a present embodiment, as seen from a front surface direction (a direction A in Fig. 1).
Fig. 4 is an overall configuration diagram of the interior of the ophthalmic device 1 of the present embodiment, as seen from a right side surface direction (a direction B in Fig. 1).
Fig. 5 is an overall configuration diagram of the interior of the ophthalmic device 1 of the present embodiment, as seen from above (a direction C in Fig. 1).
Fig. 6 is a diagram showing an example of an anterior ocular segment image 110 of a right eye of a subject.
Fig. 7 is a schematic diagram showing an eye position before a display of a fixation target 31K with respect to the subject's eye is switched to a non-display in a heterophoria examination.
Fig. 8 is a schematic diagram showing the eye position after a sufficient amount of time has elapsed from switching of the fixation target 31K with respect to the subject's eye from the display to the non-display, in a heterophoria examination.
Fig. 9 is a diagram showing an example of an eye position change graph of one eye.
Fig. 10 is a diagram in which the eye position change graph of the one eye is compared with an optical characteristic change graph, in the same time band.
Fig. 11 is a histogram showing a measurement frequency for each value of eye position measurement results.
Fig. 12 is a schematic diagram showing eye positions of a left eye EL and a right eye ER, in a state in which the fixation target 31K is displayed with respect to both eyes of the subject when the left eye EL is deviated due to strabismus.
Fig. 13 is a diagram showing changes in the eye position of the subject with concomitant strabismus, when the display of the fixation target 31K with respect to the right eye ER is switched from the state shown in Fig. 12 to the non-display.
Fig. 14 is a diagram showing a change in the eye position of the subject with non-comitant strabismus, when the display of the fixation target 31K with respect to the right eye ER is switched from the state shown in Fig. 12 to the non-display.
Fig. 15 is a schematic diagram showing changes in the eye position of the subject with uniocular strabismus, when the non-display of the fixation target 31K with respect to the right eye ER is switched from the state shown in Fig. 13 to the display.
Fig. 16 is a diagram showing an example of an eye position change graph for both eyes.

### DETAILED DESCRIPTION

Hereinafter, one exemplary embodiment of the present disclosure will be described with reference to the drawings. As an example, in the present embodiment, an ophthalmic device 1 is exemplified that is capable of implementing both a subjective measurement and an objective measurement of an optical characteristic (the refractive power, for example) of a subject's eye, in addition to an objective examination of a state relating to an eye position of the subject's eye. However, at least a portion of the technology illustrated in the present embodiment may be applied to an ophthalmic device that implements only the objective examination of the state relating to the eye position, for example. At least a portion of the technology illustrated in the present embodiment may be applied to an ophthalmic device that is not provided with a configuration to implement one of the subjective measurement and the objective measurement of the optical characteristic, for example. Of the visual functions of the subject, the ophthalmic device may be configured to be able to carry out an examination of functions other than the function relating to the eye position and the optical characteristic.

### Overall configuration

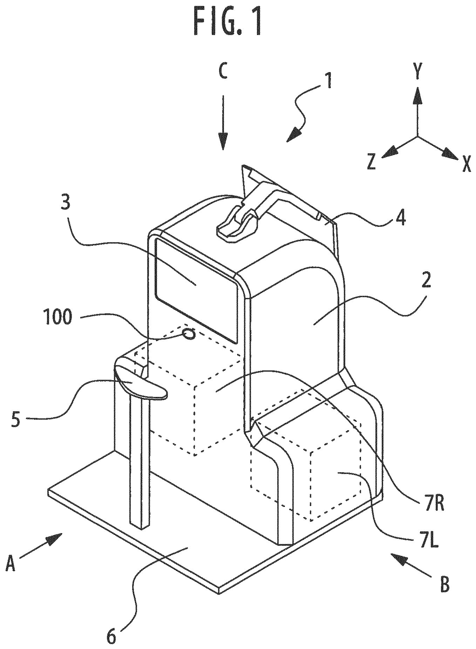

An overall configuration of the ophthalmic device 1 of the present embodiment will be explained with reference to Fig. 1. The ophthalmic device 1 of the present embodiment includes a housing 2, an exhibition window 3, a touch panel (an operation unit and a display unit) 4, a chin support 5, a base 6, a photographing optical system 100, and the like. The housing 2 internally houses various members. For example, a measurement unit 7 (a left eye measurement unit 7L and a right eye measurement unit 7R) to be described below is provided inside the housing 2. The exhibition window 3 is used to display a visual target with respect to the subject. For example, a visual target luminous flux is emitted from the left eye measurement unit 7L and the right eye measurement unit 7R and is projected onto the subject's eye via the exhibition window 3.

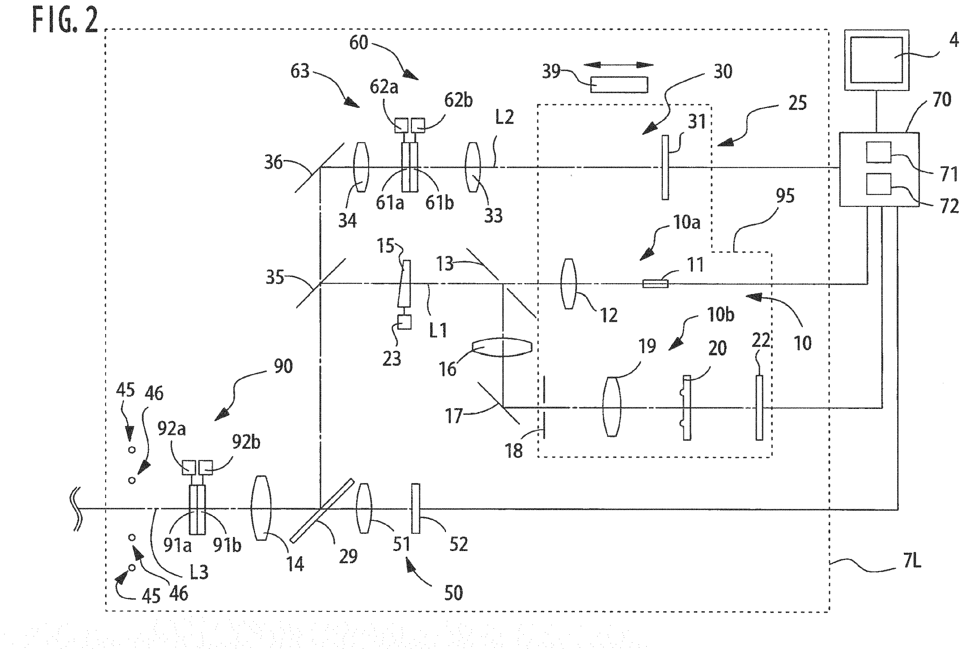

As well as displaying an image, the touch panel 4 is operated by a user. Specifically, in the present embodiment, the touch panel 4 is used as an operation unit that is operated to input various commands by the user (a tester, for example) and a display unit (a display) that displays an image. The operation unit and the display unit may be provided separately from each other. The operation unit outputs, to a control unit 70 to be described below (refer to Fig. 2), a signal corresponding to an input operation command. For example, at least one of a mouse, a joystick, a keyboard, and the like may be used as the operation unit. The display unit may be built into a main body of the ophthalmic device 1, or may be provided separately from the ophthalmic device 1. For example, various data (an eye position change graph, an eye position displacement amount, the presence/absence of strabismus, the presence/absence of heterophoria, the type of strabismus, and the like, which will be described below, for example) may be display on a display of a personal computer (hereinafter referred to as a "PC") connected to the ophthalmic device 1. A plurality of display units may be used together.

The chin support 5 supports the chin of the subject. By the chin of the subject being placed on the chin support 5, a distance between the subject's eye and the ophthalmic device 1 can be maintained to be constant, and movement of the subject's face can be suppressed. In place of the chin support 5, a head support, a face support, or the like may be used. The chin support 5 and the housing 2 are fixed to the base 6. The photographing optical system 100 includes an imaging element and a lens (not shown in the drawings). The photographing optical system 100 can photograph the face of the subject.

### Measurement unit

The configuration of the measurement unit 7 will be explained with reference to Fig. 2. In the present embodiment, the configuration of the left eye measurement unit 7L and the configuration of the right eye measurement unit 7R are substantially the same. Thus, an explanation will be made below of the left eye measurement unit 7L, and an explanation of the right eye measurement unit 7R will be omitted. The left eye measurement unit 7L includes a subjective measurement unit 25, an objective measurement unit 10, a first index projecting optical system 45, a second index projecting optical system 46, and an anterior ocular segment photographing unit 50.

### Subjective measurement unit

The subjective measurement unit 25 is used to measure the optical characteristics of the subject's eye in accordance with a response from the subject. In other words, using the subjective measurement unit 25, the optical characteristics of the subject's eye are subjectively measured. In the present embodiment, as an example, of the optical characteristics (ocular refractivity, contrast sensitivity, binocular function, and the like) of the subject's eye, the ocular refractivity is measured using the subjective measurement unit 25. The subjective measurement unit 25 of the present embodiment includes a light projecting optical system (visual target projecting system) 30, a corrective optical system 60, and a compensating optical system 90.

The light projecting optical system 30 projects the visual target luminous flux toward the subject's eye. The light projecting optical system 30 of the present embodiment includes a display 31, a projection lens 33, a projection lens 34, a reflecting mirror 36, a dichroic mirror 35, a dichroic mirror 29, and an objective lens 14. After being emitted from the display 31, the visual target luminous flux passes through the optical elements of the projection lens 33, the projection lens 34, the reflecting mirror 36, the dichroic mirror 35, the dichroic mirror 29, and the objective lens 14, in that order, and is projected onto the subject's eye.

An examination target such as a Landolt ring visual target, a fixation target for fixing the subject's eye, and the like are displayed on the display 31, for example. The fixation target is used at the time of an eye position measurement and at the time of an objective measurement to be described below, for example. The visual target luminous flux emitted from the display 31 is projected toward the subject's eye. The display 31 and the light projecting optical system 30 of the present embodiment are an example of a fixation target display unit that displays the fixation target with respect to the subject's eye. The fixation target display unit 30, 31 of the present embodiment can change a position of the fixation target displayed with respect to the subject's eye. More specifically, the position of the fixation target displayed with respect to the subject's eye is changed by changing a position of the fixation target displayed on the display 31. However, the method of changing the position of the fixation target may be changed. For example, the position of the fixation target may be changed by driving an optical member (a prism, a scanner, or the like, for example) on a display optical path of the fixation target. A display device, such as an LCD or the like, may be used as the display 31. The configuration and the method adopted to display the fixation target with respect to the subject's eye may be changed. For example, in the present embodiment, a luminous flux of the fixation target is projected separately onto the left eye and the right eye of the subject. However, the ophthalmic device 1 may display the single fixation target with respect to both the left eye and the right eye. The ophthalmic device 1 may display the fixation target with respect to the subject's eye by causing the fixation target provided on a visual target plate to be positioned on the optical path. The method of changing the position of the fixation target may be selected as appropriate in accordance with the configuration of a fixation target display unit.

The corrective optical system 60 includes an astigmatism correction optical system 63 and a driving mechanism 39. The astigmatism correction optical system 63 is disposed between the projection lens 33 and the projection lens 34. In the present embodiment, the astigmatism correction optical system 63 is used to correct the cylindrical power, the cylindrical axis, and the like of the subject's eye. For example, the astigmatism correction optical system 63 includes two positive cylindrical lenses 61a and 61b having equal focal distances. The cylindrical lenses 61a and 61b are driven, respectively, by rotary mechanisms 62a and 62b, and rotate independently around an optical axis L2. The configuration of the astigmatism correction optical system 63 may be changed. For example, the cylindrical power and the like may be corrected by inserting and removing a corrective lens into and from the optical path of the light projecting optical system 30.

The driving mechanism 39 includes a motor and a slide mechanism, and moves the display 31 in the direction of the optical axis L2. For example, by moving the display 31 at the time of the subjective measurement, the display position (the display distance) of the visual target with respect to the subject's eye is optically changed. As a result, the spherical power is corrected. By moving the display 31 at the time of the objective measurement, fogging is applied to the subject's eye. The configuration to correct the spherical power may be changed. For example, the spherical power may be corrected by inserting and removing an optical element into and from the optical path. The spherical power may be corrected by moving, in an optical axis direction, the lens disposed on the optical path.

In the present embodiment, the corrective optical system 60 that corrects the spherical power, the cylindrical power, and the cylindrical axis is exemplified. However, the corrective optical system may correct another optical characteristic (a prism value, or the like). By correcting the prism value, even if the subject's eye is an eye with heterophoria, the visual target luminous flux can be appropriately projected onto the subject's eye.

In the present embodiment, the astigmatism correction optical system 63 that corrects the cylindrical power and the cylindrical axis, and the driving mechanism 39 that corrects the spherical power are provided separately from each other. However, the spherical power, the cylindrical power, and the cylindrical axis may be corrected using the same configuration. For example, the spherical power, the cylindrical power, and the cylindrical axis may be corrected using an optical system that modulates a wave surface. A lens disc on which a plurality of optical elements are disposed on the same circumference, and an actuator that rotates the lens disc, may be used as the corrective optical system. The plurality of optical elements may include at least one of a spherical lens, a cylindrical lens, a dispersing prism, and the like. In this case, by rotating the lens disc and switching the optical element positioned on the optical axis L2, various optical characteristics can be corrected. The optical element positioned on the optical axis L2 may be rotated by the actuator. The optical element positioned on the optical axis L2 is, for example, at least one of a cylindrical lens, a cross cylinder lens, a rotary prism, and the like.

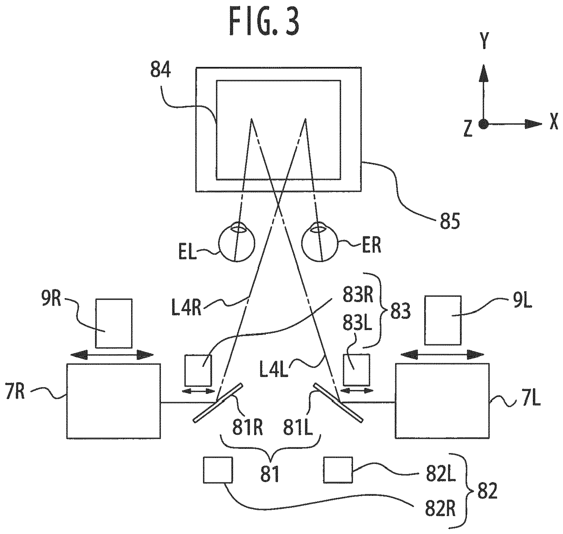

The compensating optical system 90 is disposed between the objective lens 14 and a deflecting mirror 81 to be described below (refer to Fig. 3). For example, the compensating optical system 90 may be used to correct an optical aberration occurring in the subjective measurement unit 25. The compensating optical system 90 may be used to correct an astigmatism in the optical aberration. The compensating optical system 90 of the present embodiment includes two positive cylindrical lenses 91a and 91b having equal focal distances. The cylindrical lenses 91a and 92a are driven, respectively, by rotary mechanisms 92a and 92b, and rotate independently around an optical axis L3. The compensating optical system 90 can correct the astigmatism by adjusting the cylindrical power and the cylindrical axis. The configuration of the compensating optical system 90 may be changed. For example, the optical aberration may be corrected by inserting and removing a compensating lens into and from the optical path. The corrective optical system 60 may include the functions of the compensating optical system 90. In this case, the corrective optical system 60 is driven while taking into account an astigmatism amount in addition to the cylindrical power and the cylindrical axis.

### Objective measurement unit

The objective measurement unit 10 is used to objectively measure the optical characteristic of the subject's eye. The objective measurement unit 10 may measure at least one of ocular refractivity, ocular axial length, a cornea shape, and the like, for example, as the optical characteristic of the subject's eye. As an example, in the present embodiment, the explanation will be made exemplifying the objective measurement unit 10 to measure the ocular refractivity of the subject's eye.

The objective measurement unit 10 includes a projection optical system (light projecting optical system) 10a, a light-receiving optical system 10b, and the compensating optical system 90. For example, the projection optical system 10a of the present embodiment projects spot-shaped measurement light onto the ocular fundus of the subject's eye via a center portion of the pupil of the subject's eye. The light-receiving optical system 10b of the present embodiment extracts reflected light of the measurement light reflected from the ocular fundus in a ring shape via a peripheral portion of the pupil, and captures a ring-shaped ocular fundus reflected image, using a two-dimensional imaging element 22.

The projection optical system 10a of the present embodiment includes a measurement light source 11, a relay lens 12, a hole mirror 13, a prism 15, a drive unit (motor) 23, the dichroic mirror 35, the dichroic mirror 29, and the objective lens 14. The prism 15 is a luminous flux deflecting member. The drive unit 23 drives the prism 15 to rotate around an optical axis L1. The light source 11 is conjugate with the ocular fundus of the subject's eye. A hole portion of the hole mirror 13 is conjugate with the pupil of the subject's eye. The prism 15 is disposed in a position separated from a position conjugate with the pupil of the subject's eye, and causes a luminous flux passing through the prism 15 to be eccentric with respect to the optical axis L1. The configuration of the luminous flux deflecting member may be changed. For example, in place of the prism 15, a plane-parallel plate disposed obliquely with respect to the optical axis L1 may be used as the luminous flux deflecting member.

The dichroic mirror 35, the optical axis L2 of the subjective measurement unit 25, and the optical axis L1 of the objective measurement unit 10 are coaxial with each other. The beam splitter 29 reflects the luminous flux of the subjective measurement unit 25 and the luminous flux of the objective measurement unit 10, and guides the luminous fluxes to the subject's eye.

The light-receiving optical system 10b shares the objective lens 14, the dichroic mirror 29, the dichroic mirror 35, the prism 15, and the hole mirror 13 with the projection optical system 10a. The light-receiving optical system 10b includes a relay lens 16, a mirror 17, a light-receiving aperture 18, a collimator lens 19, a ring lens 20, and the two-dimensional imaging element 22 on the optical path in the reflection direction of the hole mirror 13. The light-receiving aperture 18 and the two-dimensional imaging element 22 are conjugate with the ocular fundus of the subject's eye. The ring lens 20 includes a lens portion formed in a ring shape, and a shielding portion provided in a region other than the lens portion. The ring lens 20 is conjugate with the pupil of the subject's eye. An output from the two-dimensional imaging element 22 is input into the control unit 70.

The reflected light of the measurement light that is emitted from the projection optical system 10a and reflected by the ocular fundus of the subject's eye is reflected by the dichroic mirror 29, is further reflected by the dichroic mirror 35, and is guided to the light-receiving optical system 10b. Further, the dichroic mirror 29 allows anterior ocular segment photographing light and alignment light to be described below to pass through, thus guiding these lights to the anterior ocular segment photographing unit 50.

In the present embodiment, the measurement light source 11 of the projection optical system 10a and the light-receiving aperture 18 of the light-receiving optical system 10b, the collimator lens 19, the ring lens 20, and the two-dimensional imaging element 22 can be integrally moved in an optical axis direction. For example, in the present embodiment, a drive unit 95 that includes the display 31, the measurement light source 11 of the projection optical system 10a, the light-receiving aperture 18 of the light-receiving optical system 10b, the collimator lens 19, the ring lens 20, and the two-dimensional imaging element 22 is integrally moved in the direction of the optical axis L1, by the drive mechanism 39. At least one of the above multiple configurations may be driven by a configuration other than the drive mechanism 39. The drive unit 95 is moved in the optical axis direction such that an outer ring luminous flux is incident onto the two-dimensional imaging element 22 in each of meridian directions. In other words, by a portion of the objective measurement unit 10 being moved in the direction of the optical axis L1 in accordance with a spherical refractive error (a spherical refractive power) of the subject's eye, the spherical refractive error is corrected, and the measurement light source 11, the light-receiving aperture 18, and the two-dimensional imaging element 22 are conjugate with the ocular fundus of the subject's eye. A movement position of the drive unit 95 is detected by a potentiometer (not shown in the drawings). The hole mirror 13 and the ring lens 20 are disposed so as to be conjugate with the pupil of the subject's eye at a certain magnification, irrespective of a movement amount of the drive unit 95.

In the present embodiment, the measurement light emitted from the measurement light source 11 passes through the relay lens 12, the hole mirror 13, the prism 15, the dichroic mirror 35, the dichroic mirror 29, and the objective lens 14, and then forms a spot-shaped point light source image on the ocular fundus of the subject's eye. During that time, a pupil projection image (the luminous flux projected onto the pupil) of the hole portion of the hole mirror 13 is eccentrically rotated at high speed by the prism 15 rotating around the optical axis L1. The spot light source image projected onto the ocular fundus is reflected and dispersed and emitted from the subject's eye. The light emitted from the subject's eye is condensed by the objective lens 14, and is once more condensed at the position of the light-receiving aperture 18, via the dichroic mirror 29, the dichroic mirror 35, the prism 15, the hole mirror 13, the relay lens 16, and the mirror 17. Following that, a ring-shaped image is formed on the two-dimensional imaging element 22 by the collimator lens 19 and the ring lens 20.

The prism 15 is disposed on a shared optical path of the projection optical system 10a and the light-receiving optical system 10b. Thus, the projected light (the measurement light) emitted from the projection optical system 10a, and the light reflected from the ocular fundus pass both pass through the prism 15. As a result of this, the light reflected from the ocular fundus is scanned in reverse as if there was no eccentricity between the light projected onto the pupil and the reflected light. In the present embodiment, the compensating optical system 90 is shared by the objective measurement unit 10 and the subjective measurement unit 25. The compensating optical system used by the objective measurement unit 10 and the compensating optical system used by the subjective measurement unit 25 may be provided separately.

The configuration of the objective measurement unit 10 of the present embodiment may be changed. For example, the objective measurement unit may include a configuration in which a ring-shaped measurement index is projected from around the pupil onto the ocular fundus, the light reflected from the ocular fundus is extracted from the center portion of the pupil, and a ring-shaped ocular fundus reflected image is received by the two-dimensional imaging element 22. The objective measurement unit may include a Shack Hartman sensor, or may include a phase-difference configuration that projects a slit.

### First index projecting optical system and second index projecting optical system

The first index projecting optical system 45 and the second index projecting optical system 46 are disposed between the compensating optical system 90 and the deflecting mirror 81 (refer to Fig. 3), for example. However, the first index projecting optical system 45 and the second index projecting optical system 46 may be changed. The first index projecting optical system 45 includes an infrared light source disposed in a ring shape around the optical axis L3. The first index projecting optical system 45 emits near infrared light to project an alignment marker onto the cornea of the subject's eye. The second index projecting optical system 46 includes a ring-shaped infrared light source disposed in a position different from the infrared light source of the first index projecting optical system 45 (In Fig. 2, for convenience, only part (a cross-section) of the ring-shaped infrared light sources of the first index projecting optical system 45 and the second index projecting optical system 46 is illustrated.). In the present embodiment, the first index projecting optical system 45 projects an infinity alignment marker onto the cornea of the subject's eye. The second index projecting optical system 46 projects an infinite alignment marker onto the cornea of the subject's eye. The alignment light emitted from the second index projecting optical system 46 is also used as the anterior ocular segment photographing light for photographing the anterior ocular segment of the subject's eye using the anterior ocular segment photographing unit 50. The light sources of the first index projecting optical system 45 and the second index projecting optical system 46 are not limited to being the ring-shaped light source, and may be configured by a plurality of spotlight sources, a line-shaped light source, or the like.

### Anterior ocular segment photographing unit

The anterior ocular segment photographing unit 50 shares the objective lens 14 and the dichroic mirror 29 with the subjective measurement unit 25 and the objective measurement unit 10. The anterior ocular segment photographing unit 50 includes a photographic lens 51, and a two-dimensional imaging element 52. The two-dimensional imaging element 52 has a photographic surface disposed in a position substantially conjugate with the anterior ocular segment of the subject's eye. An output from the two-dimensional imaging element 52 is input into the control unit 70. The two-dimensional imaging element 52 captures an anterior ocular segment image of the subject's eye by receiving invisible light (near infrared light in the present embodiment). The anterior ocular segment photographing unit 50 also captures the alignment marker image formed on the cornea of the subject's eye by the first index projecting optical system 45 and the second index projecting optical system 46. The position of the alignment marker image is detected by the control unit 70.

The configuration of the anterior ocular segment photographing unit 50 may be changed. For example, in the present embodiment, the anterior ocular segment of the left eye is photographed by the anterior ocular segment photographing unit 50 of the left eye measurement unit 7L and the anterior ocular segment of the right eye is photographed by the anterior ocular segment photographing unit 50 of the right eye measurement unit 7R. In other words, the left eye and the right eye of the subject are separately photographed by the separate anterior ocular segment photographing units 50. However, the left eye and the right eye of the subject may be photographed by the single anterior ocular segment photographing unit. For example, both the left eye and the right eye of the subject may be photographed by the photographing optical system 100 described above (refer to Fig. 1).

### Control unit

The control unit 70 includes a CPU (processor) 71, a non-volatile memory 72, a RAM, a ROM, and the like. The CPU 71 performs control of the ophthalmic device 1. The non-volatile memory 72 is a non-transitory storage medium that can hold storage content even when a power source supply is cut off. For example, the non-volatile memory 72 may be at least one of a hard disk drive, a flash ROM, a removable USB memory, or the like, for example. The non-volatile memory 72 stores various data (data of an eye position change graph, data of an eye position displacement amount, data showing the presence/absence of heterophoria, data showing the presence/absence of strabismus, data showing a type of the strabismus, and the like, to be described below, for example) generated by the CPU 71. Further, the non-volatile memory 72 stores a control program used to control the ophthalmic device 1. The RAM temporarily stores various pieces of information. The ROM stores various programs, initial values, and the like. In the present embodiment, operations of the ophthalmic device 1 are controlled by the single CPU 71 provided inside the ophthalmic device 1. However, a control unit that performs control of the ophthalmic device 1 may be provided in a device (a PC or the like, for example) different from the ophthalmic device 1. The operations of the ophthalmic device 1 may be controlled by a plurality of control units.

### Internal configuration of ophthalmic device

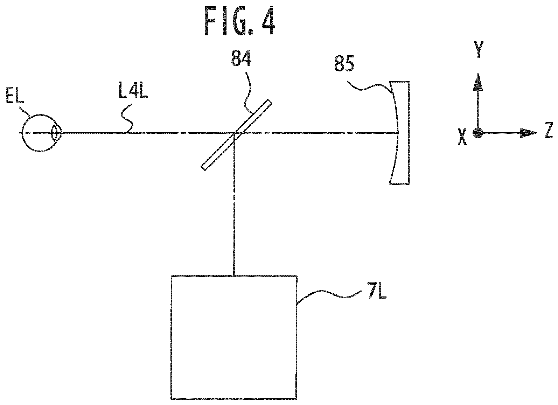

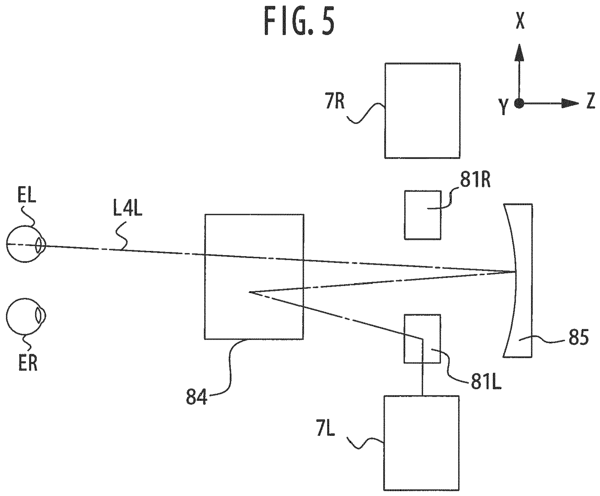

The internal configuration of the ophthalmic device 1 of the present embodiment will be explained with reference to Fig. 3 to Fig. 5. Fig. 3 is an overall configuration view of the interior of the ophthalmic device 1 of the present embodiment, as seen from a front surface direction (a direction A in Fig. 1). Fig. 4 is an overall configuration view of the interior of the ophthalmic device 1 of the present embodiment, as seen from a right side surface direction (a direction B in Fig. 1). Fig. 5 is an overall configuration view of the interior of the ophthalmic device 1 of the present embodiment, as seen from above (a direction C in Fig. 1). For convenience, an optical axis showing the reflection of a half mirror 84 (refer to Fig. 4 and Fig. 5) is not illustrated in Fig. 3. In Fig. 4 and Fig. 5, for convenience, only the optical axis of the left eye measurement unit 7L is illustrated.

The ophthalmic device 1 of the present embodiment includes the measurement unit 7 (the left eye measurement unit 7L and the right eye measurement unit 7R), a movement unit 9 (a left movement unit 9L and a right movement unit 9R), the deflecting mirror 81 (a left deflecting mirror 81L and a right deflecting mirror 81R), a drive unit 82 (a left drive unit 82L and a right drive unit 82R), a drive unit 83 (a left drive unit 83L and a right drive unit 83R), the half mirror 84, and a concave mirror 85. However, the configuration of the ophthalmic device 1 may be changed. For example, part of the above-described configuration may be omitted.

The left movement unit 9L can move the left eye measurement unit 7L in the left-right direction (an X direction). The right movement unit 9R can move the right eye measurement unit 7R in the X direction. As a result of the left eye measurement unit 7L and the right eye measurement unit 7R being moved, a distance between the left eye measurement unit 7L and the deflecting mirror 81L and a distance between the right eye measurement unit 7R and the deflecting mirror 81R are changed. In this way, the display position of the visual target luminous flux in the front-rear direction (a Z direction) is changed. Thus, as a result of the left eye measurement unit 7L and the right eye measurement unit 7R being moved, adjustment can be performed such that an image of the visual target luminous flux corrected by the corrective optical system 60 (refer to Fig. 2) is formed on the ocular fundus of the subject's eye.

The left deflecting mirror 81L is disposed on an optical path between the corrective optical system 60 of the right eye measurement unit 7L and the left eye of the subject. The right deflecting mirror 81R is disposed on an optical path between the corrective optical system of the right eye measurement unit 7R and the right eye of the subject. The left deflecting mirror 81L and the right deflecting mirror 81R are preferably positioned in a position conjugate with the pupil of the subject's eye. The left deflecting mirror 81L reflects the luminous flux emitted from the left eye measurement unit 7L and guides the luminous flux to a left eye EL, and reflects the reflected light reflected by the left eye EL and guides the reflected light to the left eye measurement unit 7L. Similarly, the right deflecting mirror 81R reflects the luminous flux emitted from the right eye measurement unit 7R and guides the luminous flux to a right eye ER, and reflects the reflected light reflected by the right eye ER and guides the reflected light to the right eye measurement unit 7R. Another deflecting member (at least one of a prism, a lens, and the like, for example) may be used in place of the deflecting mirrors 81.

The left drive unit 82L drives the left deflecting mirror 81L and the right drive unit 82R drives the right deflecting mirror 81R. The drive units 82 rotate the deflecting mirrors 81. More specifically, the drive units 82 of the present embodiment can rotate the deflecting mirror 81 around a rotation axis that extends in the horizontal direction (the X direction), and can also rotate the deflecting mirrors 81 around a rotation axis that extends in the vertical direction (a Y direction). The deflecting mirror 81 may have a single rotation axis. Further, each of the left deflecting mirror 81L and the right deflecting mirror 81R may include a plurality of mirrors having rotation axes extending in different directions. By the deflecting mirror 81 being rotated, an apparent luminous flux for forming an image in front of the subject's eye can be deflected. As a result, a formation position of the image is optically corrected.

The left drive unit 83L drives the left deflecting mirror 81L and the right drive unit 83R drives the right deflecting mirror 81R. The drive units 83 can move the deflecting mirrors 81 in the X direction. As a result of the left deflecting mirror 81L and the right deflecting mirror 81R being moved, a distance between the left deflecting mirror 81L and the right deflecting mirror 81R is changed. For example, the CPU 71 changes the distance between the left deflecting mirror 81L and the right deflecting mirror 81R in accordance with a distance between the pupils of the subject, and can thus adjust a distance in the X direction between the optical path for the left eye and the optical path for the right eye.

The concave mirror 85 guides the visual target luminous flux that has passed through the corrective optical system 60 to the subject's eye, and forms the image of the visual target luminous flux in front of the subject's eye. Further, the light emitted from the objective measurement unit 10 and reflected by the subject's eye is reflected by the concave mirror 85 and is guided to the light-receiving optical system 10b of the objective measurement unit 10. In the present embodiment, the concave mirror 85 is shared by the left eye measurement unit 7L and the right eye measurement unit 7R. However, a concave mirror disposed on the optical path for the left eye and a concave mirror disposed on the optical path for the right eye may be separately provided. An optical member configured by at least one of a lens, a half-mirror, and the like may be used in place of the concave mirror 85. A configuration may be adopted in which the reflected light of the measurement light from the objective measurement unit 10 is guided to the light-receiving optical system 10b without passing through the concave mirror 85.

Hereinafter, an optical path of the subjective measurement by the subjective measurement unit 25 will be explained. The visual target luminous flux emitted from the measurement unit 7 is reflected by the deflecting mirror 81 toward the half mirror 84, then is reflected by the half mirror 84 toward the concave mirror 85. The visual target luminous flux reflected by the concave mirror 85 reaches the subject's eye after passing through the half mirror 84. The concave mirror 85 reflects the visual target luminous flux used in the subjective measurement as a substantially parallel luminous flux. Thus, in the subject's eye, it looks as if the visual target image is present further away than an actual distance from the subject's eye to the display 31. The visual target image is formed on the ocular fundus of the subject's eye, taking a spectacle wearing position of the subject's eye (a position approximately 12 mm to the front of the apex of the cornea, for example) as a reference. The subject responds to the tester while viewing the visual target in natural vision. The optical characteristics of the subject's eye are subjectively measured by performing correction using the corrective optical system 60 until the subject can see the visual target properly.

Next, an optical path of the objective measurement using the objective measurement unit 10 will be explained. The measurement light emitted from the measurement unit 7 is reflected by the deflecting mirror 81 toward the half mirror 84, and then is reflected by the half mirror 84 toward the concave mirror 85. The measurement light reflected by the concave mirror 85 reaches the subject's eye after passing through the half mirror 84 and forms an image on the ocular fundus of the subject's eye. At this time, the pupil projection image (the luminous flux projected onto the pupil) of the hole portion of the hole mirror 13 is eccentrically rotated by the prism 15. The light of the image formed on the ocular fundus of the subject's eye is reflected and dispersed and emitted from the subject's eye, and returns to the hole mirror 13 along the optical path along which the measurement light passed. The reflected light is reflected by the hole mirror 13, is once more condensed at the opening of the light-receiving aperture 18, and is formed as a substantially parallel luminous flux (when the subject has normal vision) by the collimator lens 19. After that, the reflected light is extracted as the ring-shaped luminous flux by the ring lens 20, and is received by the two-dimensional imaging element 22. The optical characteristics of the subject's eye are objectively measured by analyzing a ring image photographed by the two-dimensional imaging element 22.

### Measurement of eye position

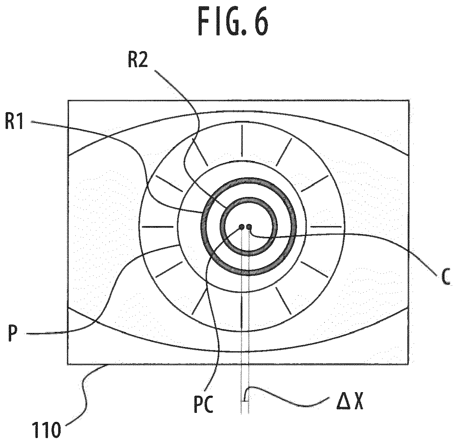

An example of a method for measuring the eye position (the direction in which the subject's eye is facing) will be explained with reference to Fig. 6. As well as measuring the eye position of the subject's eye, the ophthalmic device 1 of the present embodiment can generate eye position state information indicating an eye position state. The ophthalmic device 1 of the present embodiment can perform the measurement of the eye position of the subject's eye in parallel with the objective measurement of the optical characteristics of the subject's eye. Further, the ophthalmic device 1 can measure the eye position of each of the left eye and the right eye of the subject in parallel (simultaneously, for example), and can measure the eye position of just one of the left eye and the right eye.

The ophthalmic device 1 measures the eye position of the subject's eye on the basis of an anterior ocular segment image 110 (refer to Fig. 6) photographed by the anterior ocular segment photographing unit 50 (refer to Fig. 2). A ring marker image R1, a ring marker image R2, and a pupil P are shown in the anterior ocular segment image 110 of the right eye shown in Fig. 6. The ring marker image R1 is shown by the infrared light source of the first index projecting optical system 45. The ring marker image R2 is shown by the infrared light source of the second index projecting optical system 46. In the example shown in Fig. 6, the ring marker image R2 is positioned further to the inside than the ring marker image R1.

For example, the CPU 71 of the ophthalmic device 1 of the present embodiment detects the position of the pupil P and the visual target image (the ring marker image R2, for example) by performing image processing on the photographed anterior ocular segment image 110. The detection of the position of the pupil P may be performed by detecting a rise and fall in the luminance, and detecting an edge position of the pupil P, for example. The detection of the position of the marker image is performed in a similar manner. The CPU 71 detects a pupil center position PC on the basis of the position of the pupil P. The pupil center position PC may be detected by calculating a position of a center of the substantially circular pupil P, for example. Further, the CPU 71 detects a corneal apex position C on the basis of the position of the marker image. The corneal apex position C may be detected by calculating a position of a center of the ring marker image R2, for example. The corneal apex position C may be detected on the basis of the position of the ring marker image R1, or may be detected on the basis of the positions of both the ring marker image R1 and the ring marker image R2. Further, even when the plurality of spot-shaped light sources or the like are used in place of the ring-shaped light source, the corneal apex position C may be detected by calculating a position of a center of the plurality of marker images.

In the present embodiment, the CPU 71 measures a displacement amount ΔX between the pupil center position PC and the corneal apex position C, and the direction of the displacement, as the eye position of the subject's eye. The larger the displacement in a visual axis direction of the subject's eye with respect to a photography optical axis of the anterior ocular segment photographing unit 50, the larger the displacement amount ΔX is likely to be. Thus, the eye position is appropriately measured by calculating the displacement amount ΔX. The measured eye position may not be an absolute value. However, in this case also, by continuously measuring the eye position, a change in the eye position can be appropriately ascertained.

The method for measuring the eye position may be changed. For example, only one of the displacement amount ΔX and the direction of the displacement may be measured as the eye position. The eye position may be measured on the basis of only one of the position of the pupil P and the corneal apex position C. The eye position may be measured on the basis of the edge position of the pupil P instead of the pupil center position PC. The eye position may be measured on the basis of other information (information about the distance between the pupils of the left eye and right eye, and the like, for example). When measuring the eye position while taking the direction of the displacement into account, the direction taken into consideration may be a two-dimensional direction (the up-down direction and the left-right direction), or may be a one-dimensional direction (the up-down direction, the left-right direction, or a diagonal direction, for example).

### Heterophoria examination on basis of eye position measurement result of examination target eye

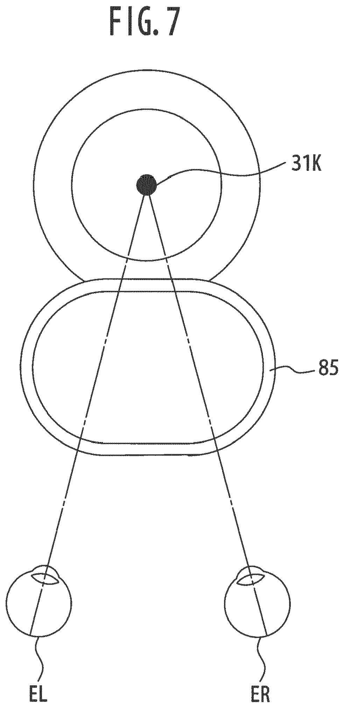

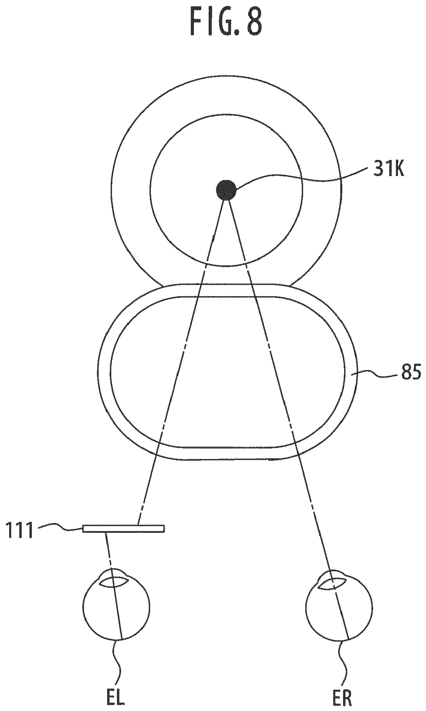

An example of a method for examining for heterophoria using the measurement result of the eye position for an examination target eye that is one of the left eye and the right eye will be explained with reference to Fig. 7 and Fig. 8. The ophthalmic device 1 of the present embodiment can perform the heterophoria examination for the subject. The heterophoria examination exemplified below is performed using the anterior ocular segment image of only the examination target eye. The heterophoria examination can be performed using the anterior ocular segment image of both the left eye and the right eye, and this content will be described below. Fig. 7 and Fig. 8 are diagrams schematically showing an optical axis and the like of a luminous flux of a fixation target 31K at the time of the heterophoria examination, and the reflection and the like of the luminous flux of the fixation target 31K by the concave mirror 85 is not illustrated. Further, Fig. 7 and Fig. 8 are schematic diagrams of a case in which, of the left eye EL and the right eye ER of the subject, the left eye EL is the examination target eye.

First, as shown in Fig. 7, the ophthalmic device 1 displays the fixation target 31K with respect to both the left eye EL and the right eye ER of the subject. In a state shown in Fig. 7, a line of sight of the left eye EL and a light of sight of the right eye ER are both aligned with the optical axis of the luminous flux of the fixation target 31K, and there is no displacement in the eye position. In other words, the subject illustrated in Fig. 7 does not have strabismus (at least no constant strabismus).

Next, as shown in Fig. 8, the display of the fixation target 31K with respect to the left eye EL that is the examination target eye is stopped (namely, the display of the fixation target 31K with respect to the left eye EL is switched to a non-display). When the left eye EL exhibits exophoria, after a sufficient amount of time has elapsed from switching from the display of the fixation target 31K, the line of sight of the left eye EL is oriented outward. The ophthalmic device 1 can detect that heterophoria is present in the left eye EL on the basis of the fact that the measurement result of the eye position of the left eye EL that is the examination target eye has changed. In other words, the ophthalmic device 1 can objectively perform the heterophoria examination of the examination target eye on the basis of the eye position of the examination target eye (the left eye EL) measured before the switching of the display of the fixation target 31K (the state shown in Fig. 7), and the eye position of the examination target eye measured after the switching of the display (the state shown in Fig. 8).

After displaying the fixation target 31K with respect to the left eye and the right eye so that the fixation target 31K seems to be close, the ophthalmic device 1 can also perform a near heterophoria examination by stopping the display of the fixation target 31K with respect to the examination target eye.

Further, in Fig. 7 and Fig. 8, the example is exemplified in which the examination target eye exhibits exophoria. However, examinations for esophoria, hyperphoria, hypophoria, and the like can also be objectively performed using the ophthalmic device 1.

The ophthalmic device 1 can also perform the eye position other than the eye position examination shown in Fig. 7 and Fig. 8. For example, the ophthalmic device 1 may switch the display state of the fixation target 31K with respect to the examination target eye (one of the left eye EL and the right eye ER) from the non-display to the display. The ophthalmic device 1 may generate the eye position state information on the basis of the eye position of the examination target eye measured before the switching of the display of the fixation target 31K (namely, during which the fixation target 31K is not displayed) and the eye position of the examination target eye measured after the switching of the display (namely, after the display of the fixation target 31K is started). Further, of the left eye and the right eye, the ophthalmic device 1 may generate the eye position state information as the examination target eye of the eye on the opposite side to the eye for which the display and non-display of the fixation target 31K is switched.

### Method for switching display of fixation target

An example of a method for switching the display and the non-display of the fixation target 31K with respect to the subject's eye will be explained. For example, as shown in Fig. 8, by inserting and removing a visible light shielding member (an IR filter or the like, for example) 111 in front of the subject's eye (in the present embodiment, at a position between the display window 3 (refer to Fig. 1) of the housing 2 and the subject's eye), the display and non-display of the fixation target 31K with respect to the subject's eye is switched. The visible light shielding member 111 allows the anterior ocular segment photographing light (the non-visible infrared light in the present embodiment), which is used by the anterior ocular segment photographing unit 50 to photograph the anterior ocular segment, to pass through, and blocks the visible light that displays the fixation target 31K. In this case, the anterior ocular segment photographing unit 50 can photograph the anterior ocular segment of the subject's eye appropriately, through the state before and after the switching of the display of the fixation target 31K. Further, when the display of the fixation target 31K is stopped, the visible light shielding member 111 is inserted in front of the subject's eye. Thus, a possibility is low that the subject's eye pays attention to a part of the ophthalmic device 1 after the display is stopped. As a result, the fixing of the subject's eye can be appropriately released after the display of the fixation target 31K is stopped.

The visible light shielding member 111 may be manually inserted into and removed from the position in front of the subject's eye by the tester. Alternatively, the ophthalmic device 1 may include an insertion/removal drive unit that causes the visible light shielding member 111 to be inserted into and removed from the display optical path of the fixation target 31K between the subject's eye and the housing 2. The insertion/removal drive unit may be provided in the housing 2, the chin support 5, or the like, for example. The CPU 71 may switch between the display and the non-display of the fixation target 31K with respect to the subject's eye, by controlling the driving of an actuator (a motor or a solenoid, for example) of the insertion/removal drive unit. A semi-transparent member that allows only a part of the visible light to pass through may be used in place of the visible light shielding member 111.

The method for switching between the display and the non-display of the fixation target 31K may be changed. For example, the CPU 71 may switch between the display and the non-display of the fixation target 31K by switching between displaying and erasing the fixation target 31K on the display 31. The CPU 71 may switch between the display and the non-display of the fixation target by causing the actuator to drive the visual target plate on which the fixation target 31K is provided. Additionally, the CPU 71 may stop the display of the fixation target 31K by blocking the display luminous flux of the fixation target 31K inside the ophthalmic device. The display and the non-display may be switched by a polarizing member provided on the display optical path of the fixation target 31K.

### Acquisition of fixation target display switch timing

In the present embodiment, the CPU 71 can acquire a display switch timing at which the display and the non-display of the fixation target with respect to the subject's eye is switched. For example, the CPU 71 may acquire the display switch timing by receiving input of a command from a user specifying the display switch timing. The specification command of the display switch timing may be input by operating an operation unit (the touch panel 4, for example), or may be input by voiced command. For example, when the display and the non-display of the fixation target is switched by manually inserting and removing the visible light shielding member 111 in front of the eye, the user may input the specification command at the timing at which the visible light shielding member 111 is inserted or removed. In this case, the CPU 71 may acquire the timing at which the specification command is input as the display switch timing. The user may input the specification command after the display switch timing.

The CPU 71 may acquire the display switch timing by detecting, using image processing or the like on the anterior ocular segment image 110, a timing at which a strength of the anterior ocular segment photographing light received by the anterior ocular segment photographing unit 50 changes due to the insertion or removal of the visible light shielding member 111, the semi-transparent member, or the like. The CPU 71 may detect the timing at which the visible light shielding member 111 is inserted in front of or removed from in front of the eye (namely, the display switch timing), on the basis of the image photographed by the photographing optical system 100. Alternatively, the ophthalmic device 1 may include a sensor that detects that the visible light shielding member 111, the semi-transparent member, or the like is inserted into or removed from the optical path of the fixation target. In this case, the CPU 71 may acquire the display switch timing on the basis of a detection result by the sensor.

Further, when the display and the non-display of the fixation target is switched by controlling the driving of the actuator (the actuator of the insertion/removal unit, the actuator that drives the visual target plate, or the like, for example), the CPU 71 may acquire, as the display switch timing, a timing at which the actuator is driven to switch between the display and the non-display. When the display and the non-display of the fixation target is switched by controlling the display or the erasure of the fixation target on the display 31, the CPU 71 may acquire, as the display switch timing, a timing at which the display or the erasure of the fixation target is switched.

### Generation of eye position change graph data

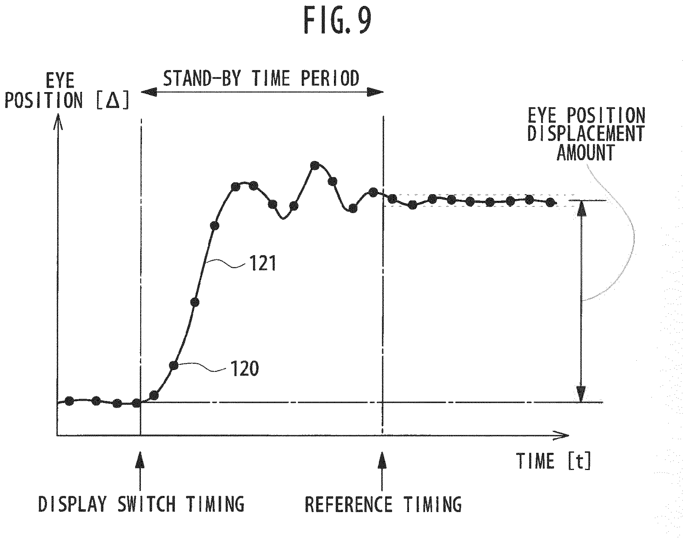

Data of an eye position change graph that can be generated by the CPU 71 will be explained with reference to Fig. 9. In the present embodiment, the CPU 71 can generate the data of the eye position change graph on the basis of eye position measurement results of a plurality of timings measured on the basis of the anterior ocular segment image 110. The data of the eye position change graph is one type of the eye state information indicating the state of the eye position of the examination target eye. The CPU 71 generates the data of the eye position change graph illustrated in Fig. 9 on the basis of the plurality of eye position measurement results measured intermittently at timings of each of a predetermined time period (each one frame of a moving image of the anterior ocular segment image 110, for example). A control unit that measures the eye position, and a control unit that generates the data of the eye position change graph on the basis of the measurement results may be different from each other.

The CPU 71 may display the eye position change graph on a display unit (the touch panel 4 or the like, for example). Further, the CPU 71 may output the generated data of the eye position change graph to an external device, or may output and store the generated data on a storage device (a non-volatile memory or the like, for example). The CPU 71 may display the eye position change graph on the display unit in real time during the examination of the eye position. The CPU 71 may generate the data of the eye position change graph after the examination, on the basis of a plurality of the anterior ocular segment images 110 photographed continuously during the examination.

The data of the eye position change graph illustrated in Fig. 9 includes data of a plurality of points 120 at which a plurality of eye measurement results with respect to one eye are plotted for individual measurement timings. The data of the eye position change graph illustrated in Fig. 9 also includes data of an approximation curve 121 generated on the basis of the plotted plurality of points 120. However, the CPU 71 may generate only one of the data of the plurality of points 120 and the data of the approximation curve 121. Data of another format of graph (a bar graph or the like, for example) may be generated.

In the present embodiment, the CPU 71 generates the data of the eye position change graph on the basis of an eye position measurement result at a timing before the display switch timing of the fixation target and an eye position measurement result at a timing after the display switch timing. In other words, the CPU 71 of the present embodiment generates at least the data of the eye position change graph after the display switch timing of the fixation target. Thus, time variations of the eye position resulting from the switching of the display and non-display of the fixation target can be appropriately ascertained using the eye position change graph. When the data of the eye position change graph of the examination target eye is generated for various types of examination, such as the heterophoria examination and the like, the tester can appropriately perform various types of diagnosis of the examination target eye using the eye position change graph.

The CPU 71 may detect a timing at which the subject's eye blinks, by processing the anterior ocular segment image 110 photographed by the anterior ocular segment photographing unit 50. The CPU 71 may include, in the data of the eye position change graph, information about the timing at which the subject's eye blinks. The CPU 71 may display, on the display unit, the timing at which the subject's eye blinks, together with the eye position change graph. The eye position just after the eye has blinked is likely to be unstable. Thus, the tester can more appropriately ascertain the state relating to the eye position, by viewing the eye position change graph while ascertaining the timing at which the eye has blinked.

### Display of anterior ocular segment image of eye position measurement timing

The CPU 71 can store, in a storage device, data of the anterior ocular segment image 110 used in the measurement of the eye position. The CPU 71 receives input of a command specifying a photography timing of the anterior ocular segment image 110 photographed in the past. The CPU 71 can display, on the display unit, the anterior ocular segment image 110 photographed at the specified photography timing. Thus, the user can easily compare the eye position measurement result of a desired timing with the anterior ocular segment image 110 used in that measurement. The input of the command specifying the photography timing may be performed by operating the operation unit, or may be performed by a voice command or the like. When the eye position change graph is displayed on the display unit, the user may input the command specifying the photography timing by specifying the desired timing on the eye position change graph. In this case, the eye position measurement result and the anterior ocular segment image 110 can be even more easily compared.

### Calculation of eye position displacement amount

The CPU 71 can generate (calculate) as the eye position state information, a displacement amount between the eye position measurement result at a timing (hereinafter referred to as a "pre-switch timing") before the display switch timing of the fixation target and the eye position measurement result at a timing (hereinafter referred to as a "post-switch timing") after the display switch timing. Thus, an eye position displacement amount resulting from the switching of the display and the non-display of the fixation target can be appropriately ascertained. Fig. 9 shows an example of the calculated eye position displacement amount. The eye position displacement amount may be calculated during the eye position examination, or may be calculated after the eye position examination.

### Reference timing

As shown in Fig. 9, a timing at which a stand-by time period has elapsed from the display switch timing of the fixation target is allocated as a reference timing. Among the timings after the switch, the CPU 71 of the present embodiment calculates the eye position displacement amount on the basis of the eye position measurement results of timings after the switch from the reference timing onward. For example, as shown by the eye position change graph illustrated in Fig. 9, after gradually changing from the display switch timing of the fixation target, the eye position when the heterophoria or the like is present tends to stabilize along with the elapsing of time. Thus, by using the eye position measurement results after the reference timing, the accuracy of the calculated eye position displacement amount can be improved.

A length of the stand-by time period from the display switch timing of the fixation target to the reference timing may be set in advance. In this case, the length of the stand-by time period is set to a length that is equal to or more than a time period required for the eye position of the examination target eye to become stable from the display switch timing ("five seconds" or the like, for example). As a result, the eye position displacement amount is calculated on the basis of the eye position that is in a stable state.

### Specification of reference timing by user

The CPU 71 can set the reference timing in accordance with a command input by the user. Thus, the user can set a desired timing as the reference timing, in accordance with various conditions (experience, a state of the subject's eye, and the like). The CPU 71 may cause the user to specify the reference timing by causing the user to specify the length of the stand-by time period from the display switch timing of the fixation target to the reference timing. Further, during the eye position examination, the CPU 71 may set a timing at which the command is input as the reference timing.

Further, the CPU 71 may cause the user to specify the reference timing in a state in which the eye position change graph (refer to Fig. 9) is displayed on the display unit. In this case, the user can specify the appropriate timing as the reference timing, while ascertaining a state of changes in the eye position using the eye position change graph. The CPU 71 may specify the reference timing by causing the user to specify a desired timing on the eye position change graph.

When the timing specified by the user is set as the reference timing, a period may be provided in which the setting of the reference timing is prohibited. For example, a period from the display switch timing of the fixation target to the elapse of the minimum stand-by time period required for the eye position of the examination target eye to become stable (one second from the display switch timing, for example) may be designated as the period in which the setting of the reference timing is prohibited. In this case, the possibility can be reduced of the eye position displacement amount being calculated on the basis of the eye position in an unstable state.

### Automatic setting of reference timing

The CPU 71 may automatically set the reference timing. Hereinafter, methods used to automatically set the reference timing will be illustrated.

First, a method will be explained to set the reference timing on the basis of a plurality of the eye position measurement results. The CPU 71 can detect a timing (hereinafter referred to as an "eye position stability timing") at which the eye position becomes stable after the display switch timing, on the basis of the eye position measurement results of a plurality of timings after the display switch timing of the fixation target. The CPU 71 can set the eye position stability timing as the reference timing.

A specific method for detecting the eye position stability timing from the plurality of eye position measurement results may be selected as appropriate. For example, the CPU 71 may detect a fluctuation in the measurement results, from the eye position measurement results of the plurality of timings after the switch. The CPU 71 may detect, as the eye position stability timing, a timing at which the detected fluctuation becomes equal to or less than a threshold value. For example, at least one of a standard deviation of the plurality of measurement results within a unit period of time, a difference between a maximum value and a minimum value of the plurality of measurement results within the unit period of time, a frequency of the varying measurement results, and the like may be adopted as the fluctuation in the measurement results. The CPU 71 may output, as the eye position state information, information about the fluctuation in the eye position measurement results. The CPU 71 may generate the data of the approximation curve 121 (refer to Fig. 9) showing a relationship between the eye position and time, from the eye position measurement results of the plurality of timings after the switch. The CPU 71 may detect, as the eye position stability timing, a timing at which the inclination of the approximation curve 121 becomes equal to or less than a threshold value.

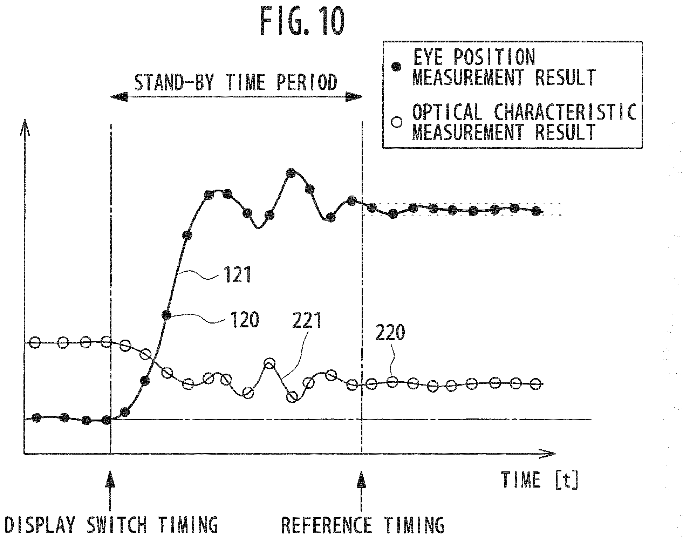

Next, a method for specifying the reference timing on the basis of the objective measurement result of the optical characteristic of the examination target eye will be explained with reference to Fig. 10. In Fig. 10, an eye position change graph and an optical characteristic change graph of the examination target eye in the same time band are compared. The optical characteristic change graph is a graph showing a relationship between time and the optical characteristic of the examination target eye objectively measured at a plurality of timings by the objective measurement unit 10. For example, in Fig. 10, the refractivity of the examination target eye is used as the objectively measured optical characteristic. The data of the optical characteristic change graph illustrated in Fig. 10 includes data of a plurality of points 220 at which a plurality of measurement results of the optical characteristic are plotted for individual measurement timings. The data of the optical characteristic change graph illustrated in Fig. 10 also includes data of an approximation curve 221 generated on the basis of the plotted plurality of points 220. However, only one of the data of the plurality of points 220 or the data of the approximation curve 221 may be generated, or data of another format of graph may be generated. The CPU 71 may output the data of the optical characteristic change graph, or may cause the optical characteristic change graph to be displayed on the display unit.

As shown in Fig. 10, when the eye position of the examination target eye becomes stable, the optical characteristic of the examination target eye is also more likely to become stable. Thus, a timing at which the optical characteristic of the examination target eye becomes stable may be detected as the eye position stability timing. The CPU 71 may detect the eye position stability timing on the basis of the objective measurement results of the optical characteristic, among the plurality of timings after the display switch timing of the fixation target. The CPU 71 may set the detected eye position stability timing as the reference timing.

A specific method for detecting the eye position stability timing from the plurality of measurement results of the optical characteristic may be selected as appropriate. For example, the CPU 71 may detect a fluctuation in the measurement results, from the measurement results of the optical characteristic of the plurality of timings after the switch. The CPU 71 may detect, as the eye position stability timing, a timing at which the detected fluctuation becomes equal to or less than a threshold value. Similarly to the fluctuations in the eye position measurement results, at least one of the standard deviation, the difference between a maximum value and a minimum value, the frequency, and the like may be adopted as the fluctuation in the measurement results of the optical characteristic. The CPU 71 may output information about the fluctuation in the measurement results of the optical characteristic. The CPU 71 may detect, as the eye position stability timing, a timing at which the inclination of the approximation curve 221 becomes equal to or less than a threshold value.

When the eye position stability timing is detected, the CPU 71 may output information about a time period from the display switch timing of the fixation target to the eye position stability timing. In this case, the user can more appropriately ascertain the statues of the eye position of the examination target eye, by referring to the output time period.

### Calculation of eye position displacement amount on basis of plurality of eye position measurement results

From the eye position measurement results of the plurality of timings after the reference timing, the CPU 71 can identify an average value of the measurement results, a median value between the maximum value and the minimum value, or a most frequent value that is a most frequently measured value. The CPU 71 can calculate a displacement amount between the identified value and the eye position measurement result before the display switch timing of the fixation target. In this case, the eye position displacement amount can be calculated while further suppressing an influence of the fluctuations in the eye position.

The CPU 71 may also identify the average value, the median value, or the most frequent value of the plurality of eye position measurement results for the eye position measurement result before the display switch timing of the fixation target. In this case, an influence of the fluctuations in the eye position before the display switch timing of the fixation target can also be suppressed.

### Calculation of eye position displacement amount on basis of measurement frequency

From the plurality of eye position measurement results during a time period from before and after the display switch timing of the fixation target, the CPU 71 may identify a first most frequent value and a second most frequent value. The first most frequent value is the eye position measurement result that is the most frequently measured value before the display switch timing. The second most frequent value is the eye position measurement result that is the most frequently measured value after the display switch timing. The CPU 71 may calculate a displacement amount between the first most frequent value and the second most frequent value as the eye position displacement amount.

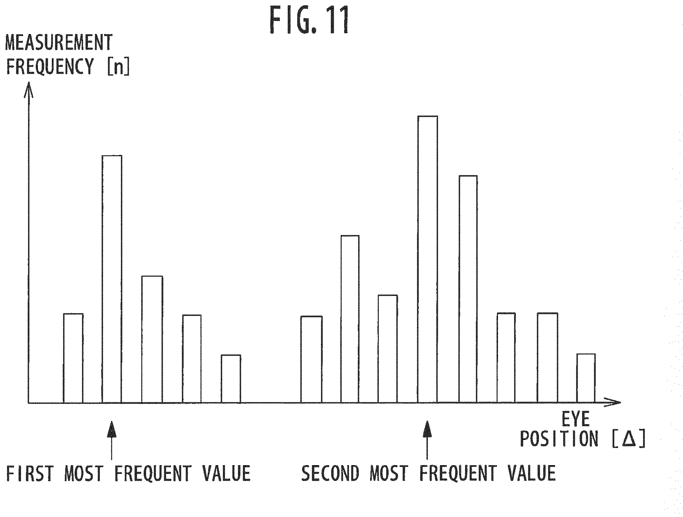

Fig. 11 is an example of a histogram showing a measurement frequency for each of values of the eye position measurement results. As shown in Fig. 11, for example, when the examination target eye exhibits heterophoria or the like, the most frequent value of the eye position measurement results before the display switch timing of the fixation target, and the most frequent value of the eye position measurement results after the display switch timing appear separately. In the heterophoria examination illustrated in Fig. 7 and Fig. 8, there is a tendency for the eye position of the examination target eye (the eye with heterophoria) during the display of the fixation target to be a smaller value than that of the eye position after the display is stopped. Thus, when the eye position examination shown in Fig. 7 and Fig. 8 is performed, of two of the most frequent values, the most frequent value for which the eye position is smaller can be determined to be the first most frequent value before the display switch timing, and the most frequent value for which the eye position is larger can be determined to be the second most frequent value after the display switch timing. The CPU 71 can calculate the eye position displacement amount for which the influence of the fluctuation in the eye position is suppressed, by calculating the displacement amount between the first most frequent value and the second most frequent value as the eye position displacement amount. In this case, the CPU 71 can calculate the appropriate eye position displacement amount, even when the above-described reference timing is not acquired.

From the eye position measurement results of a plurality of timings, the CPU 71 may generate data of a histogram showing a measurement frequency for each value of the eye position measurement results. The CPU 71 may output the data of the generated histogram. The CPU 71 may display the histogram on the display unit. When the histogram is displayed on the display unit, the CPU 71 may determine two values (a value of the eye position before the display switch timing of the fixation target, and a value of the eye position after the display switch timing) that are used to calculate the eye position displacement amount, on the basis of the input specification command. In this case, by viewing the histogram, the user can appropriately specify the two values used to calculate the eye position displacement amount.

As described above, the ophthalmic device 1 of the present embodiment generates the eye position state information on the basis of the measurement results of the eye position at the at least two timings, including the timing after the display switch timing of the fixation target and the timing before the display switch timing. Thus, a change in the state of the eye position resulting from the switching of the display and the non-display of the fixation target can be objectively and appropriately examined.

### Eye position measurement of both eyes

The ophthalmic device 1 of the present embodiment can measure both the eye position of the left eye and the eye position of the right eye of the subject, and can generate the eye position state information on the basis of the measurement results. More specifically, the ophthalmic device 1 switches the display and the non-display of the fixation target with respect to at least one of the left eye and the right eye during the examination. The ophthalmic device 1 can measure the eye position of the left eye and the eye position of the right eye at at least two timings including a timing before the display switch timing (the pre-switch timing) and a timing after the display switch timing (the post-switch timing), and can generate the eye position state information on the basis of the measurement results. In this case, in comparison to a case in which only the eye position measurement results for one eye are used, the objective examination of the state relating to the eye position can be more appropriately performed. Hereinafter, this will be explained in more detail using a specific example.

### Eye position measurement of both eyes when switching from display to non-display of fixation target with respect to one eye

For example, the ophthalmic device 1 of the present embodiment switches from a state in which the fixation target 31K is displayed with respect to both the left eye and the right eye (namely, to both eyes) to the non-display of the fixation target 31K with respect to one of the left eye and the right eye. For each of the eye for which the display of the fixation target 31K has been switched to the non-display (hereinafter referred to as "switched eye") and the eye on the opposite side to the switched eye (hereinafter referred to as the "non-switched eye"), the ophthalmic device 1 generates (calculates), as the eye position state information, the displacement amount (the eye position displacement amount) between the measurement results of the eye positions at the pre-switch timing and the post-switch timing. In this case, various diagnoses are appropriately performed on the basis of the generated eye position state information. Below, as examples, a case in which the strabismus examination is performed and a case in which the heterophoria examination is performed will be explained.

### Strabismus examination using eye position measurement of both eyes

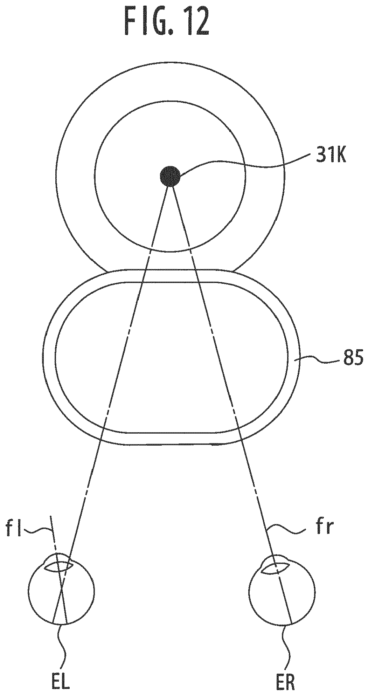

An example of the strabismus examination that can be performed using the ophthalmic device 1 of the present embodiment will be explained with reference to Fig. 12 to Fig. 14. Fig. 12 is a schematic diagram showing eye positions of the left eye EL and the right eye ER in a state in which the fixation target 31K is displayed with respect to both eyes of the subject whose left eye EL is deviated by strabismus. First, the CPU 71 displays the fixation target 31K with respect to both the left eye EL and the right eye ER of the subject. However, in the state shown in Fig. 12, a line of sight fr of the right eye ER is aligned with the optical axis of the display luminous flux of the fixation target 31K, while a line of sight frame fl of the left eye EL that is deviated by the strabismus is misaligned to the outside with respect to the optical axis of the display luminous flux of the fixation target 31K. In other words, in the example shown in Fig. 12, the left eye EL exhibits divergent strabismus.

In the state shown in Fig. 12 (namely, the state before the switching the display of the fixation target 31K with respect to the one eye to the non-display), the anterior ocular segment image 110 of the left eye EL and the right eye ER is captured at at least one timing. For example, in the present embodiment, the anterior ocular segment image 110 of the left eye EL and the right eye ER is intermittently captured a plurality of times, over a period from before to after the display switch timing of the fixation target 31K.

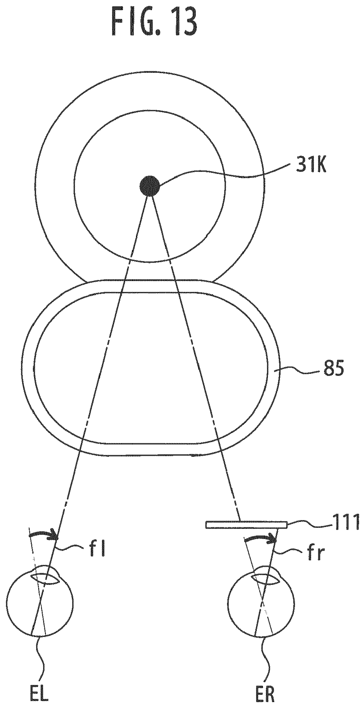

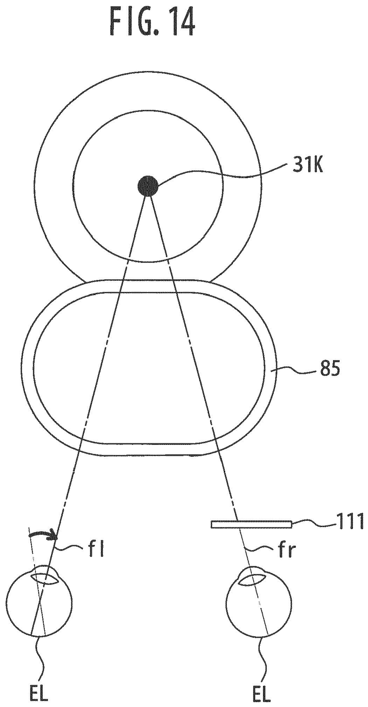

Next, the CPU 71 switches the display of the fixation target 31K with respect to the one eye (the right eye ER in Fig. 13 and Fig. 14) to the non-display. Fig. 13 and Fig. 14 show changes in the eye position of the subject with strabismus when the display of the fixation target 31K with respect to the right eye ER is switched from the state shown in Fig. 12 to the non-display. Fig. 13 is an example of a case in which the subject has concomitant strabismus and Fig. 14 is an example of a case in which the subject has non-comitant strabismus. As shown in Fig. 13 and Fig. 14, when the display of the fixation target 31K with respect to the right eye ER is changed to the non-display, the deviated left eye EL rotates and the line of sight fl of the left eye EL is aligned with the optical axis of the display luminous flux of the fixation target 31K.

The CPU 71 calculates the displacement amount between the measurement results of the eye positions for each of the right eye ER that is the switched eye and the left eye EL that is the non-switched eye, at the pre-switch timing and the post-switch timing. As shown in Fig. 12 to Fig. 14, in the state in which the non-switched eye is deviated as a result of the strabismus, when the display of the fixation target 31K with respect to the switched eye is switched to the non-display, the eye position of the non-switched eye changes. Thus, the CPU 71 determines whether the calculated eye position displacement amount of the non-switched eye is equal to or greater than a threshold value. The threshold value may be set as appropriate in accordance with various conditions (this is also applicable to threshold values in the following explanation). When the eye position displacement amount of the non-switched eye is equal to or greater than the threshold value, the CPU 71 generates the eye position state information indicating that the subject has strabismus. The user can easily ascertain, using the eye position state information, whether the subject has strabismus.

As shown in Fig. 13, when the subject has concomitant strabismus, when the display of the fixation target 31K with respect to the switched eye is switched to the non-display, the eye position of the switched eye also changes, similarly to the eye position of the non-switched eye. Thus, when the eye position displacement amount of the non-switched eye is equal to or greater than the threshold value and the eye position displacement amount of the switched eye is equal to or greater than a threshold value, the CPU 71 generates the eye position state information indicating that the subject has concomitant strabismus.

As shown in Fig. 14, when the subject has non-comitant strabismus, when the display of the fixation target 31K with respect to the switched eye is switched to the non-display, the eye position of the non-switched eye changes but the eye position of the switched eye does not easily change. Thus, when the eye position displacement amount of the non-switched eye is equal to or greater than the threshold value and the eye position displacement amount of the switched eye is less than the threshold value, the CPU 71 generates the eye position state information indicating that the subject has non-comitant strabismus.

Additionally, the CPU 71 detects, from the anterior ocular segment images 110 of the non-switched eye of the pre-switch timing and the post-switch timing, a movement direction of the eye position of the non-switched eye. The CPU 71 generates the eye position state information indicating the deviation direction of the deviated eye (one of divergent strabismus, convergent strabismus, hypertropia, and hypotropia, for example), on the basis of the detected movement direction. In the examples shown in Fig. 12 to Fig. 14, as a result of the display of the fixation target 31K being switched, the eye position of the left eye EL that is the deviated eye moves inward. Thus, the eye position state information is generated that indicates that the subject has divergent strabismus.

When only the examination is performed to determine whether the subject has strabismus, without performing the examination to determine whether it is the concomitant strabismus, the CPU 71 can generate only the eye position displacement amount of the non-switched eye, without generating the eye position displacement amount of the switched eye. In a case in which it is known in advance that the subject has strabismus or the like, the CPU 71 may generate only the eye position displacement amount of the switched eye, without generating the eye position displacement amount of the non-switched eye. In this case, it may be determined whether the strabismus is concomitant strabismus on the basis of the eye position displacement amount of the switched eye.

The CPU 71 need not necessarily generate the information indicating whether the subject has strabismus and need not necessarily generate the information indicating whether the strabismus is concomitant strabismus. For example, the CPU 71 may display the calculated eye position displacement amount on the display unit (the touch panel 4, or the like, for example). The CPU 71 may notify the calculated eye position displacement amount to the user, by voice or the like. In this case also, the user can appropriately ascertain the presence or absence of strabismus and the type of the strabismus (whether it is concomitant strabismus, for example), on the basis of the calculated eye position displacement amount.

In addition, a method similar to the above-described method may be adopted as a calculation method of the eye position displacement amount of each of the left eye and the right eye. For example, the CPU 71 may set a timing, as the reference timing, at which the stand-by time period has elapsed from the display switch timing of the fixation target 31K, and may calculate the eye position displacement amount on the basis of the eye position measurement result after the reference timing. In this case, the reference timing may be set in advance, or may be set by the user. The CPU 71 may detect the eye position stability timing from the eye position measurement results of the plurality of timings, and may set the detected eye position stability timing as the reference timing. The CPU 71 may set the reference timing on the basis of the objective measurement results of the optical characteristic of the subject's eye. The CPU 71 may identify the average value, the median value, or the most frequent value of the measurement value from the eye position measurement results of the plurality of timings, and may calculate the eye position displacement amount on the basis of the identified value. The CPU 71 may identify the first most frequent value and the second most frequent value from the plurality of eye position measurement results and may calculate the displacement amount between the first most frequent value and the second most frequent value as the eye position displacement amount. These types of processing is similar to the above-described processing and a detailed explanation thereof is therefore omitted here. The eye position displacement amount explained below may be calculated using the same methods.

### Heterophoria examination using eye position measurement of both eyes

An example of a method of a heterophoria examination using the eye position displacement amount of both eyes will be explained with reference to Fig. 7 and Fig. 8. First, as shown in Fig. 7, the CPU 71 displays the fixation target 31K with respect to both the left eye EL and the right eye ER of the subject. In the state shown in Fig. 7, the line of sight of the left eye EL and the line of sight of the right eye ER are both aligned with the optical axis of the luminous flux of the fixation target 31K, and the eye position is not likely to be displaced.

Next, as shown in Fig. 8, the CPU 71 switches the display of the fixation target 31K with respect to the one eye (the left eye EL in Fig. 8) to the non-display. Further, the CPU 71 calculates the displacement amount of the measurement results of the eye position of the pre-switch timing and the post-switch timing, for each of the left eye EL that is the switched eye and the right eye ER that is the non-switched eye.

As shown in Fig. 8, when the left eye EL that is the switched eye exhibits heterophoria, when a sufficient period of time has elapsed from the display switch timing of the fixation target 31K, the line of sight of the switched eye is in a state of being displaced from the optical axis of the display luminous flux of the fixation target 31K. Meanwhile, the line of sight of the right eye ER that is the non-switched eye remains aligned with the optical axis of the fixation target 31K even after the display switch timing of the fixation target 31K. Thus, when the eye position displacement amount of the switched eye is equal to or greater than a threshold value and the eye position displacement amount of the non-switched eye is less than a threshold value, the CPU 71 generates the eye position state information indicating that the switched eye exhibits heterophoria.

The CPU 71 may generate the eye position state information indicating the deviation direction of the heterophoria, from a movement direction of the eye position of the switched eye. The CPU 71 may display the calculated eye position displacement amount on the display unit, or may notify the eye position displacement amount to the user using voice or the like. When it is known in advance that the subject does not have strabismus (constant strabismus, for example), as described above, the determination may be made as to whether the switched eye exhibits heterophoria on the basis of the eye position displacement amount of the switched eye only.

### Eye position measurement of both eyes when switching display of fixation target with respect to one eye from non-display to display

Next, an example will be explained of the eye position measurement when switching the display of the fixation target with respect to the one eye from the non-display to the display. When the fixation target 31K is displayed with respect to one of the left eye and the right eye, the ophthalmic device 1 of the present embodiment switches the non-display of the fixation target 31K with respect to the other eye to the display. The ophthalmic device 1 can generate (calculate), as the eye position state information, the displacement amount of the measurement results of the eye position (the eye position displacement amount) between the pre-switch timing and the post-switch timing, for each of the eye for which the non-display of the fixation target 31K is switched to the display (the switched eye), and the eye on the opposite side to the switched eye (the non-switched eye). In this case, various diagnoses can be appropriately performed. Hereinafter, as an example, a case will be explained in which an examination is performed to determine whether strabismus is one of uniocular or alternating.

### Examination of uniocular heterophoria/alternating heterophoria using eye position measurement of both eyes

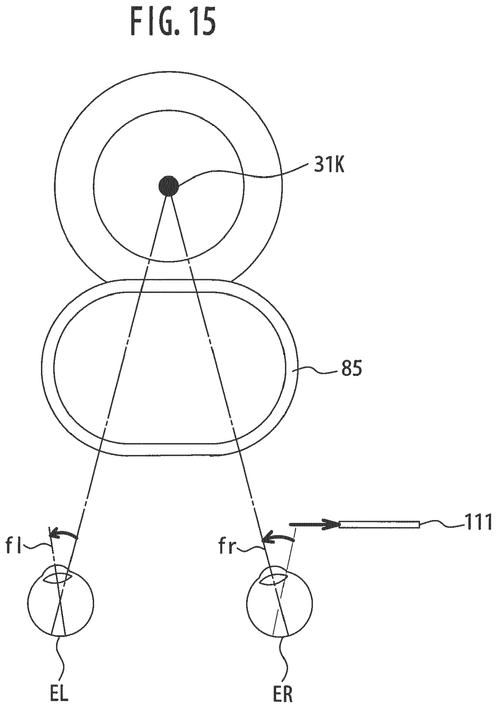

An example will be explained of an examination of uniocular heterophoria/alternating heterophoria with reference to Fig. 13 and Fig. 15. Fig. 15 is a schematic diagram showing changes in the eye position of a subject with uniocular strabismus, when the non-display of the fixation target 31K with respect to the right eye ER is switched to the display from the state shown in Fig. 13. In other words, in the present embodiment, the strabismus examination is performed in the order of Fig. 12, Fig. 13, and Fig. 15. However, the order and the like of the strabismus examination may be changed. At the point in time of the state shown in Fig. 13, it is already known that the subject has strabismus. In the example shown in Fig. 12 and Fig. 13, the left eye EL of the subject is originally deviated, and, when the display of the fixation target 31K with respect to the right eye ER is switched to the non-display, the deviated eye switches from the left eye EL to the right eye ER.

As shown in Fig. 15, the CPU 71 switches the non-display of the fixation target 31K with respect to the right eye ER to the display. As a result, a state is obtained in which the fixation target 31K is displayed with respect to both eyes. Here, in the example shown in Fig. 15, the example is of a case in which the subject has uniocular strabismus. In the case of uniocular strabismus, the line of sight fl of the originally deviated left eye EL rotates, and is once more deviate outward. Further, the line of sight fr of the right eye ER is aligned with the optical axis of the display luminous flux of the fixation target 31K. Meanwhile, although not shown in the drawings, in the case of alternating strabismus, even when the non-display of the fixation target 31K with respect to the right eye ER is switched to the display, the line of sight fl of the left eye EL remains aligned with the optical axis of the display luminous flux of the fixation target 31K.

Thus, if the eye position displacement amount of at least the non-switched eye (the left eye EL in Fig. 15) is less than a threshold value, the CPU 71 generates the eye position state information indicating that the strabismus is the alternating strabismus. When the strabismus is concomitant strabismus (refer to Fig. 15), the CPU 71 generates the eye position state information indicating that the strabismus is the alternating strabismus when the eye position displacement amount of the non-switched eye and the eye position displacement amount of the switched eye are both less than the threshold value. When the eye position displacement amount of at least the non-switched eye is equal to or greater than the threshold value, the CPU 71 generates the eye position state information indicating that the strabismus is uniocular strabismus. When the strabismus is concomitant strabismus, the CPU 71 generates the eye position state information indicating that the strabismus is uniocular strabismus when the eye position displacement amount of the non-switched eye and the eye position displacement amount of the switched eye are both equal to or greater than the threshold value. Further, the CPU 71 can further improve the examination accuracy of the uniocular heterophoria and the alternating heterophoria by taking into account the movement direction of the eye position of the non-switched eye. Specifically, when the movement direction of the eye position of the non-switched eye is the reverse of the movement direction when the display of the fixation target 31K with respect to the switched eye is started, there is an even higher possibility of alternating strabismus.

The CPU 71 may display the calculated eye position displacement amount on the display unit, or may notify the eye position displacement amount to the user using voice or the like. In this case also, the user can appropriately perform the determination of the uniocular heterophoria and the alternating heterophoria on the basis of the eye position displacement amount. In the present embodiment, the determination of the uniocular heterophoria and the alternating heterophoria can be more appropriately performed, by using both the eye position displacement amount of the non-switched eye and the eye position displacement amount of the switched eye. However, the determination of the uniocular heterophoria and the alternating heterophoria may be performed on the basis of the eye position displacement amount of the non-switched eye only.

### Position change of fixation target

As described above, the ophthalmic device 1 of the present embodiment can change the position of the fixation target 31K displayed with respect to the subject's eye. Thus, according to the ophthalmic device 1, various examinations relating to the state of the eye position can be performed.

For example, a method will be explained in which an examination to determine which of concomitant strabismus and non-comitant strabismus is present is performed by changing the position of the fixation target 31K. In the example shown in Fig. 12, the subject has strabismus and the left eye EL is deviated outward with respect to the display luminous flux of the fixation target 31K. In the example shown in Fig. 13, the right eye ER is deviated outward. As well as changing the position of the fixation target 31K displayed with respect to the subject's eye, the CPU 71 captures the anterior ocular segment image 110 of the subject's eye at at least two timings, before the position is changed and after the position is changed. The fixation target 31K whose position changes may be only the fixation target 31K that is displayed with respect to the fixated eye (the right eye ER in Fig. 12, and the left eye EL in Fig. 13), or may be the fixation target 31K that is displayed with respect to both eyes.

The CPU 71 generates (calculates) a change amount in the measurement results of the eye position before and after the position of the fixation target 31K is changed, for the eye that is deviated with respect to the fixation target 31K (the left eye EL in Fig. 12, and the right eye ER in Fig. 13). When the strabismus is concomitant strabismus, when the position of the fixation target 31K is changed, the eye position of the eye that is deviated with respect to the fixation target 31K also changes. Thus, when the calculated change amount is equal to or greater than a threshold value, the CPU 71 generates the eye position state information indicating that the strabismus is concomitant strabismus. Meanwhile, when the calculated change amount is less than the threshold value, the CPU 71 generates the eye position state information indicating that the strabismus is non-comitant strabismus. As a result, the determination as to whether the strabismus is concomitant strabismus or non-comitant strabismus can be appropriately performed. The CPU 71 may output only the change amount in the eye position of the deviated eye, without generating the information indicating whether the strabismus is concomitant strabismus.

The position of the fixation target 31K may be changed in other examinations. For example, in the strabismus examination, by performing the strabismus examination a plurality of times with respect to the same subject, it is possible to determine whether the strabismus of the subject is intermittent strabismus or constant strabismus. Here, the ophthalmic device 1 of the present embodiment performs the above-described strabismus examination a plurality of times, while changing the display position of the fixation target 31K. As a result, the determination as to whether the strabismus is intermittent or constant can be more appropriately performed. When the strabismus is alternating strabismus, the position of the fixation target 31K (the eye position) when the deviated eye alternates can be appropriately ascertained.

### Display of eye position change graph for both eyes

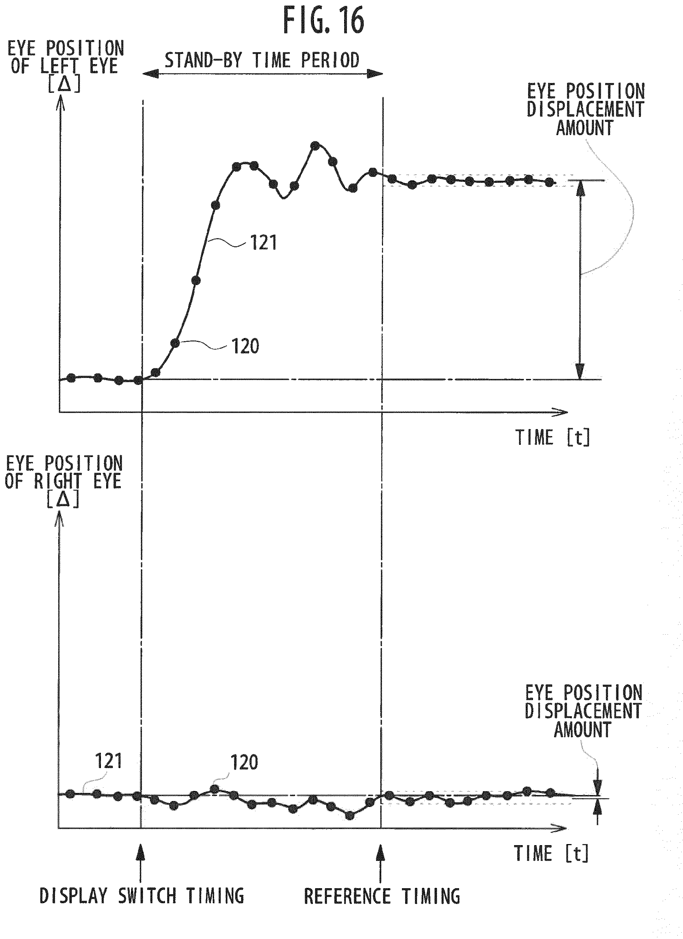

As shown in Fig. 16, the CPU 71 of the present embodiment can generate, as the eye position state information, the data of the eye position change graph showing a relationship between time and the eye positions of each of the left eye and the right eye. An eye position change graph illustrated in Fig. 16 is a graph obtained when the strabismus examination shown in Fig. 12 and Fig. 14 (namely, the strabismus examination with respect to the subject whose left eye is deviated and who has non-comitant strabismus) is performed. In the eye position change graph for both eyes, a graph of the left eye and a graph of the right eye may be individually aligned, as shown in Fig. 16, or the changes over time of the eye positions of both eyes may be shown in a single graph. The user can ascertain various states relating to the eye position by viewing the eye position change graph for both eyes. For example, by viewing the eye position change graphs shown in Fig. 16, the user can appropriately ascertain that the subject has non-comitant strabismus. A method similar to the above-described method may be adopted as a specific method to generate the data of the eye position change graph.

The CPU 71 may generate, as the eye position state information, relative information in which the measurement result of the eye position of the left eye and the measurement result of the eye position of the right eye are compared. In this case, the user can appropriately ascertain a relative relationship between the left and right eye positions. For example, the CPU 71 can generate, as the eye position state information, a difference between the eye position displacement amount of the left eye and the eye position displacement amount of the right eye. In this case, the user can appropriately ascertain the state of the left and right eye positions on the basis of the difference between the left and right eye position displacement amounts. For example, in the example shown in Fig. 16, the difference between the eye position displacement amount of the left eye and the eye position displacement amount of the right eye becomes larger. In this case, the user can appropriately ascertain, using the difference between the left and right eye position displacement amounts, that the strabismus is not concomitant strabismus and is non-comitant strabismus. The CPU 71 may calculate the eye position displacement amount by comparing the left and right eye position measurement results at a given timing.

In the present embodiment, after the display of the fixation target 31K with respect to the right eye ER is switched to the non-display, the display of the fixation target 31K with respect to the right eye ER is once more started (refer to Fig. 12 to Fig. 15). Next, after a sufficient time period has elapsed, the display of the fixation target 31K with respect to the left eye EL is switched to the non-display. After that, the display of the fixation target 31K with respect to the left eye EL is once more started, and the examination ends. However, an order of the examination may be changed. For example, the left and right order may be reversed.

The technology disclosed in the above-described embodiment is merely an example. Thus, at least a part of the technology illustrated in the above-described embodiment may be changed. For example, the data of the eye position change graph generated in the above-described embodiment is the data of a period from before the display switch timing of the fixation target to after the display switch timing. However, the CPU 71 may generate the data of the eye position change graph irrespective of the switching of the display of the fixation target. In this case also, the user can appropriately ascertain the state relating to the eye position of the examination target eye using the eye position change graph.

In the ophthalmic device 1 of the above-described embodiment, various measurements are performed in a state in which an area in front of the subject's eye is open. Specifically, in the ophthalmic device 1 of the above-described embodiment, the configuration of the corrective optical system 60 and the like is incorporated inside the housing 2 that is disposed in the position separated from the subject's eye. However, at least part of the technology illustrated in the above-described embodiment may be applied to an ophthalmic device having a different configuration to the configuration of the above-described embodiment. For example, the ophthalmic device may have a configuration in which the corrective optical system 60 and the like are incorporated in a housing that is disposed in front of the subject's eye. The ophthalmic device may be an ocular refractivity measuring device or the like that is provided with a configuration to photograph the anterior ocular segment of the subject's eye.

## Claims

1. An ophthalmic device (1) that performs an examination of a visual function of a subject, the ophthalmic device comprising:
an anterior ocular segment photographing unit (50) that captures an anterior ocular segment image of at least one of a left eye and a right eye of the subject;
a fixation target display unit (30, 31) that displays a fixation target with respect to the at least one of the left eye and the right eye of the subject; and
a processor (71) that controls the ophthalmic device,
wherein
the processor
measures an eye position of the at least one of the left eye and the right eye of the subject by processing the anterior ocular segment image captured by the anterior ocular segment photographing unit, and
generates eye position state information based on measurement results of the eye position of an examination target eye at at least two timings including a post-switch timing and a pre-switch timing, the eye position state information indicating a state of the eye position of the subject, the post-switch timing being a timing after a display switch timing at which display and non-display of the fixation target with respect to the at least one of the left eye and the right eye of the subject is switched, and the pre-switch timing being a timing before the display switch timing.

2. The ophthalmic device according to claim 1, wherein
the processor generates, as the eye position state information, at least data of an eye position change graph showing a relationship between time and the eye position of the examination target eye after the display switch timing.

3. The ophthalmic device according to claim 1 or 2, wherein
the processor generates, as the eye position state information, a displacement amount between a measurement result of the eye position of the examination target eye at at least one pre-switch timing before the display switch timing and a measurement result of the eye position of the examination target eye at at least one post-switch timing after the display switch timing.

4. The ophthalmic device according to claim 3, wherein
the processor generates the displacement amount based on a measurement result of the eye position at at least one post-switch timing after a reference timing, the reference timing being a timing at which a stand-by time period elapses from the display switch timing.

5. The ophthalmic device according to claim 4, wherein
the processor
receives input of a command specifying the reference timing, and
sets the reference timing in accordance with the input command.

6. The ophthalmic device according to claim 4, wherein
the processor
detects a timing at which the eye position of the examination target eye becomes stable after the display switch timing, based on measurement results of the eye position of the examination target eye at a plurality of the post-switch timings, and
sets the detected timing as the reference timing.

7. The ophthalmic device according to claim 4, further comprising:
an objective measurement unit (10) that objectively measures an optical characteristic of the examination target eye by emitting measurement light onto the examination target eye and receiving reflected light of the measurement light reflected by the examination target eye,
wherein
the processor
detects a timing at which the eye position of the examination target eye becomes stable after the display switch timing, based on measurement results of the optical characteristic of the examination target eye measured by the objective measurement unit at a plurality of the post-switch timings, and
sets the detected timing as the reference timing.

8. The ophthalmic device according to any one of claims 4 to 7, wherein
the processor
identifies, from a plurality of measurement results of the eye position measured at a plurality of the post-switch timings after the reference timing, one of an average value of the measurement results, a median value between a maximum value and a minimum value of the measurement results, and a most frequent value being a most frequently measured value, and
calculates the displacement amount using the identified value.

9. The ophthalmic device according to claim 1, wherein
the anterior ocular segment photographing unit captures the anterior ocular segment image of the left eye and the right eye of the subject, and
the processor
measures the eye position of each of the left eye and the right eye of the subject by processing the anterior ocular segment image captured by the anterior ocular segment photographing unit, and
generates the eye position state information based on measurement results of the eye position of each of the left eye and the right eye at the at least two timings including the pre-switch timing and the post-switch timing.

10. The ophthalmic device according to claim 9, wherein
when the display of the fixation target with respect to one of the left eye and the right eye is switched to the non-display, from a state in which the fixation target is being displayed with respect to both the left eye and the right eye, the processor generates, as the eye position state information, a displacement amount between measurement results of the eye position before the display switch timing and the eye position after the display switch timing, for each of a switched eye and a non-switched eye, the switched eye being an eye for which the display of the fixation target is switched to the non-display, and a non-switched eye being on the opposite side to the switched eye.

11. The ophthalmic device according to claim 10, wherein
the processor generates the eye position state information based on a displacement amount of the eye position of the switched eye and a displacement amount of the eye position of the non-switched eye, the eye position state information indicating at least one of presence/absence of strabismus of the subject and whether the strabismus is concomitant strabismus.

12. The ophthalmic device according to claim 10 or 11, wherein
the processor generates the eye position state information based on a displacement amount of the eye position of the switched eye and a displacement amount of the eye position of the non-switched eye, the eye position state information indicating presence/absence of heterophoria of the switched eye.

13. The ophthalmic device according to any one of claims 9 to 12, wherein
when, from a state in which the fixation target is being displayed with respect to one of the left eye and the right eye, the non-display of the fixation target is switched to the display with respect to the other eye, the processor generates, as the eye position state information, a displacement amount of measurement results of the eye position before the display switch timing and the eye position after the display switch timing, for each of a switched eye and a non-switched eye, the switched eye being an eye for which the non-display of the fixation target is switched to the display, and the non-switched eye being on the opposite side to the switched eye.

14. The ophthalmic device according to any one of claims 9 to 13, wherein
the processor generates, as the eye position state information, at least data of an eye position change graph showing a relationship between time and the eye position of each of the left eye and the right eye after the display switch timing.

15. The ophthalmic device according to any one of claims 9 to 14, wherein
the fixation target display unit is configured to change a position of the displayed fixation target, and
when the subject has strabismus, the processor generates, as the eye position state information, information indicating a change amount in measurement results of the eye position of an eye whose line of sight is deviated from a display luminous flux of the fixation target when the position of the displayed fixation target is changed.
